# EUROPEAN PATENT SPECIFICATION

(11) **EP 1 705 255 B1**
(45) Date of publication and mention of the grant of the patent: **15.06.2011**
(21) Application number: 06075400.9
(22) Date of filing: 09.11.1995
(51) Int. Cl.: C12Q 1/68, C12P 19/34, C12P 21/06, C12N 1/20, C12N 15/00, C07H 21/02, C07H 21/04, C07K 5/00, C07K 16/00, C07K 16/36, G01N 33/80

(54) **Diagnostic method and kit for determining Kell blood group genotype**
Diagnostisches Verfahren und Kit zur Bestimmung des Kell-Blutgruppengenotyps
Méthode de diagnostic et kit pour déterminer le génotype des groupes sanguins Kell

(30) Priority: 10.11.1994 US 337268; 07.06.1995 US 484570
(43) Date of publication of application: 27.09.2006
(62) Divisional of application: 95940686.9
(73) Proprietor: New York Blood Center, Inc., New York, New York 10021 (US)
(72) Inventor: Lee, Soohee, Cliffside Park, NJ 07010 (US); Redman, Colvin L., Franklin Square, NY 11010 (US)
(74) Representative: Thomas, Simon

(56) References cited:
- US-A- 5 213 963
- US-A- 5 302 512
- LEE SOOHEE ET AL: "Molecular basis of the kell (K1) phenotype." BLOOD, vol. 85, no. 4, 15 February 1995 (1995-02-15), pages 912-916, XP001147021 ISSN: 0006-4971
- LEE S ET AL: "MOLECULAR CLONING AND PRIMARY STRUCTURE OF KELL BLOOD GROUP PROTEIN" PROCEEDINGS OF THE NATIONAL ACADEMY OF SCIENCES OF THE UNITED STATES, vol. 88, no. 14, July 1991 (1991-07), pages 6353-6357, XP002240298 1991 ISSN: 0027-8424
- REDMAN C M ET AL: "COMPARISON OF HUMAN AND CHIMPANZEE KELL BLOOD GROUP SYSTEMS" TRANSFUSION (ARLINGTON), vol. 29, no. 6, 1989, pages 486-490, XP009009119 ISSN: 0041-1132
- LEE S ET AL: "MOLECULAR CHARACTERIZATION OF JSA/JSB (K6/K7), KPA/KPB/KPC (K3/K4/K21)AND ULA (K10) IN THE KELL BLOOD GROUP SYSTEM" TRANSFUSION, AMERICAN ASSOCIATION OF BLOOD BANKS, BETHESDA, MD, US, vol. 35, no. 10, October 1995 (1995-10), page S95, XP009009109 ISSN: 0041-1132
- LEE S ET AL: "Molecular basis of the K:6,-7 (Js(a+b-)) phenotype in the Kell blood group system." TRANSFUSION (BETHESDA), vol. 35, no. 10, 1995, pages 822-825, XP009009118 ISSN: 0041-1132
- RUSSO D C W ET AL: "Topology of kell blood group protein and the expression of multiple antigens by transfected cells." BLOOD, vol. 84, no. 10, 15 November 1994 (1994-11-15), pages 3518-3523, XP002240299 ISSN: 0006-4971
- LEE SOOHEE ET AL: "Organization of the gene encoding the human Kell blood group protein." BLOOD, vol. 85, no. 5, 1 March 1995 (1995-03-01), pages 1364-1370, XP002240300 ISSN: 0006-4971
- GRUNNET NIELS ET AL: "Evaluation of histo-blood group ABO genotyping in a Danish population: Frequency of a novel O allele defined as O-2." VOX SANGUINIS, vol. 67, no. 2, 1994, pages 210-215, XP009009656 ISSN: 0042-9007
- LEE S ET AL: "Point mutations characterize KEL10, the KEL3, KEL4, and KEL21 alleles, and the KEL17 and KEL11 alleles." TRANSFUSION (BETHESDA), vol. 36, no. 6, 1996, pages 490-494, XP009009079 ISSN: 0041-1132

### Sequence Listing

(1) GENERAL INFORMATION:
   (i) APPLICANT: Lee, Soohee Redman, Colvin L.
   (ii) TITLE OF INVENTION: Diagnostic Method and Kit for Determining Kell Blood Group Genotype
   (iii) NUMBER OF SEQUENCES: 65
   (iv) CORRESPONDENCE ADDRESS:
      (A) ADDRESSEE: Ronald J. Baron, Esq.
         Hoffmann & Baron (B) STREET: 350 Jericho Turnpike (C) CITY: Jericho (D) STATE: New York (E) COUNTRY: USA (F) ZIP: 11753
   (v) COMPUTER READABLE FORM:
      (A) MEDIUM TYPE: Diskette, 3.50 inch, 1.44 Mb storage
      (B) COMPUTER: IBM PC Compatible
      (C) OPERATING SYSTEM: MS-DOS
      (D) SOFTWARE: WordPerfect 6.1
   (vi) CURRENT APPLICATION DATA:
      (A) APPLICATION NUMBER: 08/484,570
      (B) FILING DATE: 07-JUN-1995
      (C) CLASSIFICATION:
   (vii) PRIOR APPLICATION DATA:
      (A) APPLICATION NUMBER: 08/337,268
      (B) FILING DATE: 11-OCT-1994
      (C) CLASSIFICATION:
   (viii) ATTORNEY/AGENT INFORMATION:
      (A) NAME: Baron, Ronald J.
      (B) REGISTRATION NUMBER: 29,281
      (C) REFERENCE/DOCKET NUMBER: 454-3 PCT
   (ix) TELECOMMUNICATION INFORMATION:
      (A) TELEPHONE: (516) 822-3550
      (B) TELEFAX: (516) 822-3582
(2) INFORMATION FOR SEQ ID NO:1:
   (i) SEQUENCE CHARACTERISTICS:
      (A) LENGTH: 26 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:1:
      ATACTGACTC ATCAGAAGTT TCAGCA 26
(3) INFORMATION FOR SEQ ID NO:2:
   (i) SEQUENCE CHARACTERISTICS:
      (A) LENGTH: 26 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:2:
      ATACTGACTC ATCAGAAGTC TCAGCA 26
(4) INFORMATION FOR SEQ ID NO:3:
   (i) SEQUENCE CHARACTERISTICS:
      (A) LENGTH: 26 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:3:
      TGGACTTCCT TAAACTTTAA CTGAAC 26
(5) INFORMATION FOR SEQ ID NO:4:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:4:
      TTTAGTCCTC ACTCCCATGC TTCC 24
(6) INFORMATION FOR SEQ ID NO:5:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:5:
      TATCACACAG GTGTCCTCTC TTCC 24
(7) INFORMATION FOR SEQ ID NO:6:
   (i) SEQUENCE CHARACTERISTICS:
      (A) LENGTH: 35 nucleotides
      (B) TYPE: nucleic acid
      (C) STRANDEDNESS: single
      (D) TOPOLOGY: linear
   (ii) MOLECULE TYPE:genomic DNA
   (iii) HYPOTHETICAL: no
   (xi) SEQUENCE DESCRIPTION: SEQ ID NO:6:
      GGAGACTTCC AAGGTCTTAG CTATCACTTA AGCAC 35
(8) INFORMATION FOR SEQ ID NO:7:
   (i) SEQUENCE CHARACTERISTICS:
      (A) LENGTH: 38 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:7:
      CTGGTTCGGC CCACCTCTGA AGGTTCCAGA ATCGATAG 38
(9) INFORMATION FOR SEQ ID NO:8:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:8:
      CTCGGCTCTT CCTCACTTTG GTCC 24
(10) INFORMATION FOR SEQ ID NO:9:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:9:
      CTCTTGGCTC CAGAGAGTTC CCAT 24
(11) INFORMATION FOR SEQ ID NO:10:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:10:
      CCCACCTTCC ATCTGTCTAT CTTC 24
(12) INFORMATION FOR SEQ ID NO:11:
   (i) SEQUENCE CHARACTERISTICS:
      (A) LENGTH: 35 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:11:
      GACTCGAGTC GACAACGTTT TTTTTTTTTT TTTTT 35
(13) INFORMATION FOR SEQ ID NO:12:
   (i) SEQUENCE CHARACTERISTICS:
      (A) LENGTH: 17 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:12:
      ATGGGGAGAC TGTCCTG 17
(14) INFORMATION FOR SEQ ID NO:13:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:13:
      CAGTCCTCCG AATCAGCTCC TAGA 24
(15) INFORMATION FOR SEQ ID NO:14:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:14:
      CTCTTGGCTC CAGAGAGTTC CCAT 24
(16) INFORMATION FOR SEQ ID NO:15:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:15:
      GAAGGTGGGG ACCAAAGTGA GGAA 24
(17) INFORMATION FOR SEQ ID NO:16:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:16:
      ACAGGGTTTG GAGCAGTCAT GGTC 24
(18) INFORMATION FOR SEQ ID NO:17:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:17:
      ATATTCCCCA CCTCCCCACA CCTG 24
(19) INFORMATION FOR SEQ ID NO:18:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:18:
      ATCTACGGTG CTCAGGCTCT CCTC 24
(20) INFORMATION FOR SEQ ID NO:19:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:19:
      GGAAGCATGG GAGTGAGGAC TAAA 24
(21) INFORMATION FOR SEQ ID NO:20:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:20:
      TGGCATCCAT GGTACCTCAT GGAA 24
(22) INFORMATION FOR SEQ ID NO:21:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:21:
      GAGGCTTTTG AAACCCCAGG ATGA 24
(23) INFORMATION FOR SEQ ID NO:22:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:22:
      TTCCCCAGCC ACCTGCCATC TCAT 24
(24) INFORMATION FOR SEQ ID NO:23:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:23:
      CCCTTTTCCA AGGGTCAGAA GCTG 24
(25) INFORMATION FOR SEQ ID NO:24:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:24:
      GGGCTTATTT GACCCCCAGA ATCT 24
(26) INFORMATION FOR SEQ ID NO:25:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:25:
      CCTAATCCCT GGATGCCTGC CTGT 24
(27) INFORMATION FOR SEQ ID NO:26:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:26:
      CAGTGAGGAC ATCTGCAGAA GAGG 24
(28) INFORMATION FOR SEQ ID NO:27:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:27:
      TCCTGTGGAC CCTCCCCCTT CAAT 24
(29) INFORMATION FOR SEQ ID NO:28:
   (i) SEQUENCE CHARACTERISTICS:
      (A) LENGTH: 25 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:28:
      GGGCGGAAGC CAAGTGCCAG CTTTT 25
(30) INFORMATION FOR SEQ ID NO:29:
   (i) SEQUENCE CHARACTERISTICS:
      (A) LENGTH: 34 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:29:
      AATAAAAGCT GGCACTTGGC TTCCGCCGGA ATTC 34
(31) INFORMATION FOR SEQ ID NO:30:
   (i) SEQUENCE CHARACTERISTICS:
      (A) LENGTH: 25 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:30:
      AATAAAAGCT GGCACTTGGC TTCCG 25
(32) INFORMATION FOR SEQ ID NO:31:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:31:
      AATAAAAGCT GGCACTTGGC TTCC 24
(33) INFORMATION FOR SEQ ID NO:32:
   (i) SEQUENCE CHARACTERISTICS:
      (A) LENGTH: 23 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:32:
      AATAAAAGCT GGCACTTGGC TTC 23
(34) INFORMATION FOR SEQ ID NO:33:
   (i) SEQUENCE CHARACTERISTICS:
      (A) LENGTH: 19 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:33:
      AATAAAAGCT GGCACTTGG 19
(35) INFORMATION FOR SEQ ID NO:34:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:34:
      AATAAAAGCT GGCACTTGGC TTCC 24
(36) INFORMATION FOR SEQ ID NO:35:
   (i) SEQUENCE CHARACTERISTICS:
      (A) LENGTH: 34 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:35:
      AATAAAAGCT GGCACTTGGC TTCCGCTTGT CTCT 34
(37) INFORMATION FOR SEQ ID NO:36:
   (i) SEQUENCE CHARACTERISTICS:
      (A) LENGTH: 35 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:36:
      AATAAAAGCT GGCACTTGGC TTCCGCTTGT CTCTT 35
(38) INFORMATION FOR SEQ ID NO:37:
   (i) SEQUENCE CHARACTERISTICS:
      (A) LENGTH: 148 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL no
   (xi) SEQUENCE DESCRIPTION: SEQ ID NO:37: 
GAAGTGCCCC TTCTCCAGGA TCAAGGAACT GGGGCGGGGG GTGTTTCCTG 50

GACCCCAGTC CTCCGAATCA GCTCCTAGAG TGGAACCAGG AAGGATTCTG 100

GAGCCACAGA AGATAGACAG ATG GTAAGTCCCC TTTTGGAGTC AGAGG 148
Met
(39) INFORMATION FOR SEQ ID NO:38:
   (i) SEQUENCE CHARACTERISTICS:
      (A) LENGTH: 128 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:38: 
CTCCTTCTCC CTCCACTCAC TTCAG GAA GGT GGG GAC CAA AGT GAG GAA 49
Glu Gly Gly Asp Gln Ser Glu Glu
1               5

GAG CCG AGG GAA CGC AGC CAG GCA GGT GGA ATG GGA ACT CTC TGG 94
Glu Pro Arg Glu Arg Ser Gln Ala Gly Gly Met Gly Thr Leu Trp
    10                  15                  20

AGC CAA GAG GTAAGTGGCC TCCTCTCCTG GGTCT 128
Ser Gln Glu
    25
(40) INFORMATION FOR SEQ ID NO:39:
   (i) SEQUENCE CHARACTERISTICS:
      (A) LENGTH: 207 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:39: 
TTTCACCTCT TGGTTCCTCC CACAG AGC ACT CCA GAA GAG AGG CTG CCC 49
Ser Thr Pro Glu Glu Arg Leu Pro
1               5

GTG GAA GGG AGC AGG CCA TGG GCA GTG GCC AGG CGG GTG CTG ACA 94
Val Glu Gly Ser Arg Pro Trp Ala Val Ala Arg Arg Val Leu Thr
    10                  15                  20

GCT ATC CTG ATT TTG GGC CTG CTC CTT TGT TTT TCT CTC CTT TGT 139
Ala Ile Leu Ile Leu Gly Leu Leu Leu Cys Phe Ser Leu Leu Cys
    25                  30                  35

TTT TCT GTG CTT TTG TTC TAC AAC TTC CAG AAC TGT GGC CCT C 182
Phe Ser Val Leu Leu Phe Tyr Asn Phe Gln Asn Cys Gly Pro
    40                  45                  50

GTAAGCAAGA TCCCAGACCC CCCAA 207
(41) INFORMATION FOR SEQ ID NO:40:
   (i) SEQUENCE CHARACTERISTICS:
      (A) LENGTH: 227 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:40: 
CCCAGCTCTG AGCTTTTCCC CACAG GC CCC TGT GAG ACA TCT GTG TGT 48
Arg Pro Cys Glu Thr Ser Val Cys
1               5

TTG GAT CTC CGG GAT CAT TAC CTG GCC TCT GGG AAC ACA AGT GTG 93
Leu Asp Leu Arg Asp His Tyr Leu Ala Ser Gly Asn Thr Ser Val
    10                  15                  20

GCC CCC TGC ACC GAC TTC TTC AGC TTT GCC TGT GGA AGG GCC AAA 138
 
Ala Pro Cys Thr Asp Phe Phe Ser Phe Ala Cys Gly Arg Ala Lys
    25                  30                  35

GAG ACC AAT AAT TCT TTT CAG GAG CTT GCC ACA AAG AAC AAA AAC    183
Glu Thr Asn Asn Ser Phe Gln Glu Leu Ala Thr Lys Asn Lys Asn
    40                  45                  50

CGA CTT CGG AGA ATA CTG G GTGAGGAAAG CAGGGTGGAA GATGC          227
Arg Leu Arg Arg Ile Leu
    55
(42) INFORMATION FOR SEO ID NO:41:
   (i) SEQUENCE CHARACTERISTICS:
      (A) LENGTH: 181 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:41: 
TTTAGTCCTC ACTCCCATGC TTCCTTTCTA G AG GTC CAG AAT TCC TGG 48
Glu Val Gln Asn Ser Trp
1               5

CAC CCA GGC TCT GGG GAG GAG AAA GCC TTC CAG TTC TAC AAC TCC 93
His Pro Gly Ser Gly Glu Glu Lys Ala Phe Gln Phe Tyr Asn Ser
            10                  15                  20

TGC ATG GAT ACA CTT GCC ATT GAA GCT GCA GGG ACT GGT CCC CTC 138
Cys Met Asp Thr Leu Ala Ile Glu Ala Ala Gly Thr Gly Pro Leu
            25                  30                  35

AGA CAA GTT ATT GAG GAG GTGAGAAAAG TTGGGATATT AACTT 181
Arg Gln Val Ile Glu Glu
            40
(43) INFORMATION FOR SEQ ID NO:42:
   (i) SEQUENCE CHARACTERISTICS:
      (A) LENGTH: 197 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:42: 
TCAGCCCCCT CTCTCTCCTT TAAAG CTT GGA GGC TGG CGC ATC TCT GGT 49
Leu Gly Gly Trp Arg Ile Ser Gly
1               5

AAA TGG ACT TCC TTA AAC TTT AAC CGA ACG CTG AGA CTT CTG ATG 94
Lys Trp Thr Ser Leu Asn Phe Asn Arg Thr Leu Arg Leu Leu Met
    10                  15                  20

AGT CAG TAT GGC CAT TTC CCT TTC TTC AGA GCC TAC CTA GGA CCT 139
Ser Gln Tyr Gly His Phe Pro Phe Phe Arg Ala Tyr Leu Gly Pro
    25                  30                  35

CAT CCT GCC TCT CCA CAC ACA CCA GTC ATC CAG GTGAGGGATG 182
His Pro Ala Ser Pro His Thr Pro Val Ile Gln
    40                  45

CACTGGCGAA GACAC 197
(44) INFORMATION FOR SEQ ID NO:43:
   (i) SEQUENCE CHARACTERISTICS:
      (A) LENGTH: 113 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:43: 
TCTCTCCAGT CTCTCTTGTG CCCAG ATA GAC CAG CCA GAG TTT GAT GTT 49
Ile Asp Gln Pro Glu Phe Asp Val
1               5

CCC CTC AAG CAA GAT CAA GAA CAG AAG ATC TAT GCC CAG GTAAG 93
Pro Leu Lys Gln Asp Gln Glu Gln Lys Ile Tyr Ala Gln
    10                  15                  20

ATGGCACATG GACAAAGGCC 113
(45) INFORMATION FOR SEQ ID NO:44:
   (i) SEQUENCE CHARACTERISTICS:
      (A) LENGTH: 239 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:44: 
TGTGACTGAC ATTTCCTTCC TCCAG ATC TTT CGG GAA TAC CTG ACT TAC 49
Ile Phe Arg Glu Tyr Leu Thr Tyr
1               5

CTG AAT CAG CTG GGA ACC TTG CTG GGA GGA GAC CCA AGC AAG GTG 94
Leu Asn Gln Leu Gly Thr Leu Leu Gly Gly Asp Pro Ser Lys Val
    10                  15                  20

CAA GAA CAC TCT TCC TTG TCA ATC TCC ATC ACT TCA CGG CTG TTC 139
Gln Glu His Ser Ser Leu Ser Ile Ser Ile Thr Ser Arg Leu Phe
    25                  30                  35

CAG TTT CTG AGG CCC CTG GAG CAG CGG CGG GCA CAG GGC AAG CTC 184
Gln Phe Leu Arg Pro Leu Glu Gln Arg Arg Ala Gln Gly Lys Leu
    40                  45                  50

TTC CAG ATG GTC ACT ATC GAC CAG CTC AAG GTGCCTGGAA 224
Phe Gln Met Val Thr Ile Asp Gln Leu Lys
    55                  60

CTGGGGGCA GAAGA 239
(46) INFORMATION FOR SEQ ID NO:45:
   (i) SEQUENCE CHARACTERISTICS:
      (A) LENGTH: 199 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:45: 
CTCAGCTTTG TGTCCCTCCT CTAAG GAA ATG GCC CCC GCC ATC GAC TGG 49
Glu Met Ala Pro Ala Ile Asp Trp
 
1                   5

TTG TCC TGC TTG CAA GCG ACA TTC ACA CCG ATG TCC CTG AGC CCT    94
Leu Ser Cys Leu Gln Ala Thr Phe Thr Pro Met Ser Leu Ser Pro
    10                  15                  20

TCT CAG TCC CTC GTG GTC CAT GAC GTG GAA TAT TTG AAA AAC ATG   139
Ser Gln Ser Leu Val Val His Asp Val Glu Tyr Leu Lys Asn Met
    25                  30                  35

TCA CAA CTG GTG GAG GAG ATG CTG CTA AAG CAG AG GTTCGCCGCA     184
Ser Gln Leu Val Glu Glu Met Leu Leu Lys Gln Arg
    40                  45                  50

GGTGGGATTG GGGAG                                              199
(47) INFORMATION FOR SEQ ID NO:46:
   (i) SEQUENCE CHARACTERISTICS:
      (A) LENGTH: 180 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:46: 
GTGTGGGTCT CTTTTGTCTC CATAG G GAC TTT CTG CAG AGC CAC ATG 47
Asp Phe Leu Gln Ser His Met
1               5

ATC TTA GGG CTG GTG GTG ACC CTT TCT CCA GCC CTG GAC AGT CAA 92
Ile Leu Gly Leu Val Val Thr Leu Ser Pro Ala Leu Asp Ser Gln
        10                  15                  20

TTC CAG GAG GCA CGC AGA AAG CTC AGC CAG AAA CTG CGG GAA CTG 137
Phe Gln Glu Ala Arg Arg Lys Leu Ser Gln Lys Leu Arg Glu Leu
        25                  30                  35

ACA GAG CAA CCA CCC ATG GTGAGGAGAG GAGCGGGTGT ATTTG 180
Thr Glu Gln Pro Pro Met
        40
(48) INFORMATION FOR SEQ ID NO:47:
   (i) SEQUENCE CHARACTERISTICS:
      (A) LENGTH: 161 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:47: 
ACTCATTCCA GCTTTGTCTC CATAG CCT GCC CGC CCA CGA TGG ATG AAG 49
Pro Ala Arg Pro Arg Trp Met Lys
1               5

TGC GTG GAG GAG ACA GGC ACG TTC TTC GAG CCC ACG CTG GCG GCT 94
Cys Val Glu Glu Thr Gly Thr Phe Phe Glu Pro Thr Leu Ala Ala
    10                  15                  20

TTG TTT GTT CGT GAG GCC TTT GGC CCG AGC ACC CGA AGT GCT 136
Leu Phe Val Arg Glu Ala Phe Gly Pro Ser Thr Arg Ser Ala
    25                  30                  35

GTATGTGAGA GCTCTTCCCA GCCCA 161
(49) INFORMATION FOR SEQ ID NO:40:
   (i) SEQUENCE CHARACTERISTICS:
      (A) LENGTH: 149 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:48: 
CTGTCCCTGG ACCTCACTCC CACAG GCC ATG AAA TTA TTC ACT GCG ATC 49
Ala Met Lys Leu Phe Thr Ala Ile
1               5

CGG GAT GCC CTC ATC ACT CGC CTC AGA AAC CTT CCC TGG ATG AAT 94
Arg Asp Ala Leu Ile Thr Arg Leu Arg Asn Leu Pro Trp Met Asn
    10                  15                  20

GAG GAG ACC CAG AAC ATG GCC CAG GAC AAG GTCAGGCCAG 134
Glu Glu Thr Gln Asn Met Ala Gln Asp Lys
    25                  30
 GCGTCCTGGC TGGTG 149
(50) INFORMATION FOR SEQ ID NO:49:
   (i) SEQUENCE CHARACTERISTICS:
      (A) LENGTH: 128 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:49: 
TAGCCTCTTC TGTGTCTCTC TCCAG GTT GCT CAA CTG CAG GTG GAG ATG 49
Val Ala Gln Leu Gln Val Glu Met
1               5

GGG GCT TCA GAA TGG GCC CTG AAG CCA GAG CTG GCC CGA CAA GAA 94
Gly Ala Ser Glu Trp Ala Leu Lys Pro Glu Leu Ala Arg Gln Glu
    10                  15                  20

TAC AAC GAT GTGGGTCCCT GTGTTTTCCA GCTCC 128
Tyr Asn Asp
    25
(51) INFORMATION FOR SEQ ID NO:50:
   (i) SEQUENCE CHARACTERISTICS:
      (A) LENGTH: 151 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:50: 
AAGTCACCTC CTGCCTCTTC CCCAG ATA CAG CTT GGA TCG AGC TTC CTG 49
Ile Gln Leu Gly Ser Ser Phe Leu
1               5

CAG TCT GTC CTG AGC TGT GTC CGG TCC CTC CGA GCT AGA ATT GTC 94
Gln Ser Val Leu Ser Cys Val Arg Ser Leu Arg Ala Arg Ile Val
10 15 25
 
CAG AGC TTC TTG CAG CCT CAC CCC CAA CAC AG GTATGACAGC AGGGG 141
Gln Ser Phe Leu Gln Pro His Pro Gln His Arg
30 35

AGACACAGGC 151
(52) INFORMATION FOR SEQ ID NO:51:
   (i) SEQUENCE CHARACTERISTICS:
      (A) LENGTH: 161 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:51: 
GAGTTCACAT GTCCTCTTCC CACAG G TGG AAG GTG TCC CCT TGG GAC 47
Trp Lys Val Ser Pro Trp Asp
1               5

GTC AAT GCT TAC TAT TCG GTA TCT GAC CAT GTG GTA GTC TTT CCA 92
Val Asn Ala Tyr Tyr Ser Val Ser Asp His Val Val Val Phe Pro
        10                  15                  20

GCT GGA CTC CTC CAA CCC CCA TTC TTC CAC CCT GGC TAT CCC AG 136
Ala Gly Leu Leu Gln Pro Pro Phe Phe His Pro Gly Tyr Pro Arg
        25                  30                  35

GTATGGGTCA CTCTGTAAGG GTAGG 161
(53) INFORMATION FOR SEQ ID NO:52:
   (i) SEQUENCE CHARACTERISTICS:
      (A) LENGTH: 118 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:52: GTCAAATAAG CCCTTGTCTC CCTAG A GCC GTG AAC TTT GGC GCT GCT 47 
Ala Val Asn Phe Gly Ala Ala
1               5

GGC AGC ATC ATG GCC CAC GAG CTG TTG CAC ATC TTC TAC CAG CTC     92
Gly Ser Ile Met Ala His Glu Leu Leu His Ile Phe Tyr Gln Leu
        10                  15                  20

T GTGGGTAACA GGGGCCACTG GGAGG                                   118
(54) INFORMATION FOR SEQ ID NO:53:
   (i) SEQUENCE CHARACTERISTICS:
      (A) LENGTH: 220 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:53: 
TGTTCTCTTG TCCCATTTTC AACAG TA CTG CCT GGG GGC TGC CTC GCC 48
Leu Leu Pro Gly Gly Cys Leu Ala
1               5

TGT GAC AAC CAT GCC CTC CAG GAA GCT CAC CTG TGC CTG AAG CGC 93
Lys Asp Asn His Ala Leu Gln Glu Ala His Leu Cys Leu Lys Arg
    10                  15                  20

CAT TAT GCT GCC TTT CCA TTA CCT AGC AGA ACC TCC TTC AAT GAC 138
His Tyr Ala Ala Phe Pro Leu Pro Ser Arg Thr Ser Phe Asn Asp
    25                  30                  35

TCC CTC ACA TTC TTA GAG AAT GCT GCA GAC GTT GGG GGG CTA GCC 183
Ser Leu Thr Phe Leu Glu Asn Ala Ala Asp Val Gly Gly Leu Ala
    40                  45                  50

ATC GCG CTG CAG GTATGCAAGT GTCAAGGGCC ACAGT 220
Ile Ala Leu Gln
    55
(55) INFORMATION FOR SEQ ID NO:54:
   (i) SEQUENCE CHARACTERISTICS:
      (A) LENGTH: 146 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:54: 
CCCTTCTCTA CCCACCCCTA CCCAG GCA TAC AGC AAG AGG CTG TTA CGG 49
Ala Tyr Ser Lys Arg Leu Leu Arg
1               5

CAC CAT GGG GAG ACT GTC CTG CCC AGC CTG GAC CTC AGC CCC CAG 94
His His Gly Glu Thr Val Leu Pro Ser Leu Asp Leu Ser Pro Gln
    10                  15                  20

CAG ATC TTC TTT CGA AGC TAT GCC CAG GTAGGCAGCG GCCACCTCCC 141
Gln Ile Phe Phe Arg Ser Tyr Ala Gln
    25                  30

GCCAC 146
(56) INFORMATION FOR SEQ ID NO:55:
   (i) SEQUENCE CHARACTERISTICS:
      (A) LENGTH: 313 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:55: 
TTCAATAACC TCTCTTCCTG CTCAG GTG ATG TGT AGG AAG CCC AGC CCC 49
Val Met Cys Arg Lys Pro Ser Pro
1               5

CAG GAC TCT CAC GAC ACT CAC AGC CCT CCA CAC CTC CGA GTC CAC 94
Gln Asp Ser His Asp Thr His Ser Pro Pro His Leu Arg Val His
    10                  15                  20

GGG CCC CTC AGC AGC ACC CCA GCC TTT GCC AGG TAT TTC CGC TGT 139
Gly Pro Leu Ser Ser Thr Pro Ala Phe Ala Arg Tyr Phe Arg Cys
    25                  30                  35

GCA CGT GGT GCT CTC TTG AAC CCC TCC AGC CGC TGC CAG CTC TGG 184
Ala Arg Gly Ala Leu Leu Asn Pro Ser Ser Arg Cys Gln Leu Trp
 
    40                  45                  50
TAACTTGGTT ACCAAAGATG CCACAGCACA GAAATATCGA CCAACACCTC 234
CCTGGTCACA TCCATGGAAT CAGAGCAAGA TTTCCTTTCT GCTTCTGTTC 284
CAAAAATAAA AGCTGGCACT TGGCTTCCG 313
(57) INFORMATION FOR SEQ ID NO:56:
   (i) SEQUENCE CHARACTERISTICS:
      (A) LENGTH: 327 nucleotides
      (B) TYPE: nucleic acid
      (C) STRANDEDNESS: single (D) TOPOLOGY: linear
   (ii) MOLECULE TYPE: genomic DNA
   (iii) HYPOTHETICAL: no
   (xi) SEQUENCE DESCRIPTION: SEQ ID NO:56: 
GTCACAGTGC AAGACAAAAG GAGCAGACCA AGGGCAAGAT TGCTTGGGGA 50
GTGAAGACTC CCTCCCTCTT CTCCCCTGAG AAGCTGAGAT AAAGGGGGAG 100
GAGAAGCCTG GGTGCCCCCC ACTGATAAGC AGGCTCCACC CAGAGGCCAG 150
TCCTGTGTGT CTGGGGACAA GGCGAAAGAG CAGCAGAAGT GCCCCTTCTC 200
CAGGATCAAG GAACTGGGGC GGGGGGTGTT TCCTGGACCC CAGTCCTCCG 250
AATCAGCTCC TAGAGTGGAA CCAGGAAGGA TTCTGGAGCC ACAGAAGATA 300
GACAGAGTGT AAGTCCCCTT TTGGAGT 327
(58) INFORMATION FOR SEQ ID NO:57:
   (i) SEQUENCE CHARACTERISTICS:
      (A) LENGTH: 45 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:57: 
TGG ACT TCC TTA AAC TTT AAC CGA ACG CTG AGA CTT CTG ATG AGT 45
Trp Thr Ser Leu Asn Phe Asn Arg Thr Leu Arg Leu Leu Met Ser
1               5                   10                  15
(59) INFORMATION FOR SEQ ID NO:58:
   (i) SEQUENCE CHARACTERISTICS:
      (A) LENGTH: 45 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:58: 
TGG ACT TCC TTA AAC TTT AAC CGA ATG CTG AGA CTT CTG ATG AGT 45
Trp Thr Ser Leu Asn Phe Asn Arg Met Leu Arg Leu Leu Met Ser
1               5                   10                  15
(60) INFORMATION FOR SEQ ID NO:59:
   (i) SEQUENCE CHARACTERISTICS:
      (A) LENGTH: 5 amino acids
      (B) TYPE: amino acid
      (D) TOPOLOGY: linear
   (ii) MOLECULE TYPE: peptide
   (v) FRAGMENT TYPE: internal fragment
   (xi) SEQUENCE DESCRIPTION: SEQ ID NO:59: 
His Glu Leu Leu His
1               5
(61) INFORMATION FOR SEQ ID NO:60:
   (i) SEQUENCE CHARACTERISTICS:
      (A) LENGTH: 5 amino acids
      (B) TYPE: amino acid
      (D) TOPOLOGY: linear
   (ii) MOLECULE TYPE: peptide
   (v) FRAGMENT TYPE: internal fragment
   (ix) FEATURE:
      (A) NAME/KEY: variable residues
      (B) LOCATION: 3-4
      (C) OTHER INFORMATION: /note= "consensus sequence found in active sites of zinc neutral peptidases"
   (xi) SEQUENCE DESCRIPTION: SEQ ID NO:60: 
His Glu Xaa Xaa His
1               5
(62) INFORMATION FOR SEQ ID NO:61:
   (i) SEQUENCE CHARACTERISTICS:
      (A) LENGTH: 26 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:61:
      ATACTGACTC ATCAGAAGTG TCAGCA 26
(63) INFORMATION FOR SEQ ID NO:62:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:62:
      TTAGTCCTCA CTCNCCATGC TTCC 24
(64) INFORMATION FOR SEQ ID NO:63:
   (i) SEQUENCE CHARACTERISTICS:
      (A) LENGTH: 22 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:63:
      TCACACAGGT GTCCTCTCTT CC 22
(65) INFORMATION FOR SEQ ID NO:64:
   (i) SEQUENCE CHARACTERISTICS:
      (A) LENGTH: 26 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:64:
      CTCACCTAGG CAGCACCAAC CCTA 24
(66) INFORMATION FOR SEQ ID NO:65:
   (i) SEQUENCE CHARACTERISTICS:
      (A) LENGTH: 25 nucleotides
      (B) TYPE: nucleic acid
      (C) STRANDEDNESS: single
      (D) TOPOLOGY: linear
   (ii) MOLECULE TYPE: genomic DNA
   (iii) HYPOTHETICAL: no
   (xi) SEQUENCE DESCRIPTION: SEQ ID NO:65:
      TTACCTGGAG GGCATGGTTG TCACT 25

## Description

### BACKGROUND OF THE INVENTION

This invention relates to a method for determining Kell blood group genotype. More particularly, the invention relates to molecular genetic methods for the determination of K6/K7, genotypes.

The Kell blood group system is a well-known but complex group of blood antigens, comprising over 20 different related antigens. Of these, the antigen K1 (K, Kell) is known to be the strongest immunogen among the 23 known phenotypes. Serologically, the K1 sublocus has an allelic relationship with a high frequency antigen K2 (k, cellano). Approximately 9% of the population has the K1 red cell phenotype, and antibodies to K1 are developed in about 5% of persons receiving a single unit of incompatible blood (Ref. 1).

Hemolytic disease of the newborn (KDN) is usually associated with maternal alloimmunization to Rh(D), but K1 incompatibilities can also cause severe hemolytic disease in newborns (Refs. 2-7). K1 sensitization from a previous pregnancy can result in HDN and complications during subsequent pregnancies if the fetus is a K1 carrier. Similar types of problems can arise in the more rare case of K2 sensitization. The identification of the fetal K1/K2 genotype would be of particular significance in situations in which the father is a K1/K2 heterozygote. Since the mother must be a homozygote in order to have been previously sensitized, there is a 50% chance that the fetus with a K:1,2 father is in danger of HDN. Because of this 50% risk, identification of the homozygosity or heterozygosity of the K1/K2 genotype of the fetus in these pregnancies becomes important in order to aid in their proper management. It is important to recognize, however, that the determination of parental genotype with respect to Kell antigens other than K1/K2 can also be of significance for managing pregnancies.

Kell inheritance is autosomal and codominant, and the gene for the Kell protein (KEL) has been mapped to chromosome 7q33 (Refs. 8-11). Kell antigens appear to be encoded in 5 sets of antithetical paired alleles expressing high and low prevalence antigens. Thus, K1 (K) and K2 (k) are products of alleles, as are K3 (Kp^{a}), K4 (Kp^{b}) and K21 (Kp^{c}) ; K6 (Js^{a}) and K7 (Js^{b}); K17 and K11; and K24 and K14. However, a number of high prevalence antigens such as K12, K13, K18 and K22 are independently expressed. Some Kell phenotypes are segregated by racial or ethnic groups. For example, K6, which was first reported in 1958, is more frequent in African-Americans, occurring in up to 19.5% in this group in a study performed in Seattle, Washington (Ref. 12). Also, K10 (Ul^{a}) is more prevalent in Finns (Ref. 13). These various relationships, and their places in the Kell system, have been established through the years by serological analyses of informative families (Refs. 14-18).

A variety of studies, including a molecular cloning, established that Kell blood group antigens are carried on a 93 kDa type II glycoprotein (Refs. 19-25) found on the surface of red blood cells (Ref. 26). The Kell protein has a short, 46 amino acid, N-terminal domain in the cytoplasm, and a large C-terminal portion, of 665 amino acids, on the external surface of the red cell. All of the carbohydrates are N-linked (Ref. 27), probably located in 5 sites, at asparagines 93, 115, 191, 345 and 627. Early biochemical studies suggested that Kell antigens reside on a protein whose conformation is largely dependent on disulfide bonds (Ref. 28). The Kell protein has 16 cysteine residues, one in the transmembrane region and 15 in the external portion (Ref. 25). Reduction of red cells by sulfhydryl reagents results in loss of Kell antigens and exposure of some neo-epitopes (Ref. 28).

The antigenic structure of the Kell protein has also begun to be mapped. A recently developed immunological test, MAIEA, which uses monoclonal antibodies to different Kell antigens, indicates that certain of the identified Kell antigens occur in spatially distinct regions of the glycoprotein (Ref. 26). For example, the K1/K2, and K6/K7 domains appear to be close together, while K3/K4 epitope is in a different location and K18 is in yet another protein domain (Ref. 29).

The determination of Kell genotype has heretofore been confined to inferences drawn from the detection and identification of Kell antigens. Such methods employ antibodies or other compounds which identify and interact with the Kell protein or portions thereof. For example, various antibodies specific for particular Kell antigens have been identified (Refs. 13, 22). Agglutination methods for detecting Kell protein and other blood group antigens are described in U.S. Patent Nos. 5,324,479, 5,302,512, 5,213,963, 4,560,647, 4,403,042, 4,358,436, and 4,148,607. These methods provide information about expressed protein profiles, not about protein molecular structure or the molecular genetic makeup of the individual. Zelinski et al. describe a method for indirectly inferring Kell genotype from linkage studies using marker genes (Ref. 30). Such methods, however, provide only limited and indirect information about Kell genotype. Generally, these methods also require obtaining blood samples from subjects being examined. Determining fetal Kell phenotype also normally requires a fetal blood sample. This involves a potentially dangerous procedure in which the fetus is susceptible to hemorrhage and possibly death. None of the methods previously described discloses any method by which Kell genotype might be determined simply and directly on the basis of the *KEL* gene structure.

As a result, there exists a need for a method of safely and conveniently detecting Kell genotype. It would also be desirable to provide a method for determining Kell genotype in a fetus without the requirement for obtaining blood samples. A test based on DNA samples taken from amniotic cells would allow the clinician to avoid the risk of harm to the fetus and to more accurately predict the potential of anti-Kell-associated HDN.

### SUMMARY OF THE INVENTION

The present invention provides a diagnostic method as claimed in claim 1 for the differential determination of Kell blood group genotype in a subject. The diagnostic method includes generating a characteristic nucleic acid product, from a source or sample of nucleic acid containing a Kell polymorphism locus, in an amount which is sufficient to enable the characterization of Kell genotype. The method may be employed to determine Kell genotype with respect to one or more Kell polymorphisms or Kell phenotypes. The method is directed to detecting the Kell polymorphism loci which determine K6/K7 genotype.

In a first preferred embodiment, the method includes:
selectively cleaving a nucleic acid sample which includes a Kell polymorphism locus, preferably genomic DNA of the subject, to provide a nucleic acid product which includes one or more nucleic acid fragments in an amount sufficient to characterize Kell genotype. The method includes selectively cleaving nucleic acid which includes the K6/K7 polymorphism locus.

The selective cleaving of DNA is accomplished by digesting the sample nucleic acid with a restriction enzyme which cleaves the DNA differentially based on a Kell polymorphism locus. Any suitable restriction enzyme may be employed as desired. In the case of the K6/K7 polymorphism locus, preferred restriction enzymes include MnII and/or DdeI. Combinations of enzymes may be employed when more than one Kell polymorphism is desired to be differentiated.

This diagnostic method preferably includes amplifying Kell DNA from a DNA sample obtained from a subject. Typically the amplification involves using a primer which amplifies only nucleic acid including the locus which determines a specific Kell polymorphism. Primers may be selected to selectively amplify DNA including particular Kell polymorphism loci, including K6/K7 polymorphism locus.

Preferably, the digesting of Kell nucleic acid by a restriction enzyme is performed following amplification of nucleic acid obtained from the subject, but the digesting may be performed preceding such amplification.

In a second preferred embodiment, the diagnostic method of the invention involves differentially amplifying a nucleic acid sample which includes a Kell polymorphism locus to provide a nucleic acid product which can be employed to characterize Kell genotype. Preferably, the sample nucleic acid operates as template permitting amplification by means of a primer which differentially amplifies nucleic acid encoding one specific allele of a set of alleles associated with a Kell polymorphism.

In the diagnostic methods of the invention which require a primer or primer set, the primer or primer set may include a single primer, or may include a plurality of primers, including, for example, a plurality of primer pairs.

Primers are included which differentially amplify nucleic acid templates containing a K6 locus and/or a K7 locus.

An especially preferred oligonucleotide primer specific for K6- and K7-containing nucleic acids has nucleotide sequences selected from the group of nucleotide sequences including: CTC ACC TAG CCA CCA CCA ACC CTA (SEQ ID No:64) and TTA CCT GGA GGG CAT GGT TGT CAC T (SEQ ID NO:65).

The diagnostic method of the invention preferably further includes the derivation of information from generated nucleic acid restriction fragments or amplification products. Preferably, the derivation of information includes separating the nucleic acid products to provide a pattern of fragments which provides specific information characterizing Kell genotype. More preferably, the separation provides a pattern of nucleic acid products which provides specific information characterizing K6/K7 genotype. In addition, it is preferred to detect some or all of the Kell nucleic acid products, such as by marking or staining one or more products. Products may be marked with a non-specific marker substance. Such substances may be used to stain some or all of the products to permit their differentiation based on the pattern of their separation. Alternatively, the detecting step may include marking one or more of the Kell nucleic acid products with one or more hybridization probes in a selective or specific fashion. Preferably, a hybridization probe(s) is employed which specifically marks one or more nucleic acid products which include a Kell polymorphism locus of interest.

The molecular genetic method of the invention generally involves obtaining nucleic acid, preferably genomic DNA, from a biological sample from a human subject or patient. A blood sample is typically preferred, but other types of tissue samples which contain erythroid tissue are useful.

The invention further provides Kell-based nucleic acid oligomers which include nucleotide sequences which are at least substantially complementary to a polymorphic region of a Kell-baned nucleic acid, such as Kell genomic DNA, mRNA or cDNA. Preferably, the oligomers of the invention are exactly complementary to the region of interest.

Moreover, the invention provides nucleic acid oligomers which include a nucleic acid sequence substantially homologous to a polymorphic region of a Kell-based nucleic acid, such as Kell genomic DNA, mRNA or cDNA. These oligomers of the invention preferably include a nucleic acid sequence which is at least in part, exactly homologous to a target region of a Kell-based nucleic acid.

The nucleic acid oligomers of the invention may be used as hybridization probes to identify the presence of target nucleic acid sequences, through binding to or hybridizing with such target sequences. Accordingly, the nucleic acid oligomers may be detectably labeled by being linked to a detectable marker moiety such as a fluorescent label, an electron dense substance, a reporter moiety, a specific or nonspecific binding moiety, or other detectable moiety such as is known in the art. Optionally, the oligomers of the invention may further include a reactive moiety permitting cross-linking with a target nucleic acid sequence. Furthermore, the oligomers of the invention may be linked to a substrate material, for example, to a gel or resin to immobilize the oligomers.

The oligomers of the invention may also be employed as amplification primers which specifically bind to and cause elongation through a region of Kell nucleic acid comprising a Kell polymorphism locus. Preferred primers bind to or cause elongation through Kell nucleic acid which contains a a K6/K7 locus.

Thus in another embodiment, the invention provides a diagnostic method for detecting alloimmunization of a patient to a Kell antigen, preferably K1 antigen. In this embodiment, the method includes obtaining a blood sample from a patient or subject, and measuring a parameter of immune reactivity of the sample with a polypeptide probe. The invention enables comparable methods for the detection of alloinmunization of a subject to other Kell antigens, including K6, K10, as well as K3 and K21.

In another embodiment, a diagnostic kit as claimed in claim 14 is provided for determining Kell blood group genotype in a sample of tissue from a patient. In this embodiment, the invention provides a diagnostic kit for determining Kell blood group genotype by detecting target nucleic acid sequences. The kit includes amplification primers. The in vitro kit further includes a container, such as a microtiter plate having a plurality of wells, having bound thereto oligonuoleotide capture probes having nucleic acid sequences substantially complementary to the target sequences.

The invention further provides expression recombinant vectors which carry Kell nucleic acid sequences. The invention provides expression vectors which include a nucleic acid sequence which encodes at least a part of the Kell protein including a part of the protein which encodes a site of Kell polymorphism. In particular, the invention provides expression vectors which K1 cDNA (not according to the invention) permitting transformation of cells to produce transformed cells or transformants which express K1 protein on their cell surfaces. Vectors are also provided which carry K6 cDNA, permitting transformation of cells to express K₆, respectively. The invention also provides a stable cell line which has been modified (i.e., transformed) to express protein on its cell surface, as well as a method for transforming a cell line to express such protein. With respect to the expression of K6, the expressed protein preferably includes the K6 domain; more preferably, the protein is K6 protein. The invention further provides a method of making an antibody specific for a Kell antigen, by inducing the generation of at least one antibody against a kell antigen expressed by a transformed cell line produced according to the invention. Polyclonal antibodies are contemplated, but the antibody is preferably monospecific, such as a monoclonal antibody or an antigen-binding region thereof.

Accordingly, as a result of the invention, there is now provided a safe and convenient diagnostic method for differentially determining the Kell genotype of patients. In particular, there is now provided a method for determining Kell genotype in a fetus in utero without requiring the taking of a blood sample. A test based on determining Kell genotype, in particular K1//K2 genotype, (not according to the invention) from DNA obtained from amniotic fluid now allows the clinician to reduce the degree of risk to the fetus being tested. Moreover, the new diagnostic method permits the accurate prediction of the potential of anti-K-associated hemolytic disease of the newborn.

These and other advantages of the present invention will be appreciated from the detailed description and examples which are set forth herein. The detailed description and examples enhance the understanding of the invention, but are not intended to limit the scope of the invention.

### BRIEF DESCRIPTION OF THE DRAWINGS

Preferred embodiments of the invention have been chosen for purposes of illustration and description, but are not intended in any way to restrict the scope of the present invention. The preferred embodiments of certain aspects of the invention are shown in the accompanying drawing, wherein:
Figure 1 shows a map of the Kell gene indicating the loci of the Kell exons, the relationships among the clones used to establish the exon locations, as well as restriction sites in the Kell gene.
Figures 2A-2D show the nucleotide sequences of the exons of the Kell gene.
Figure 3 shows the 5' flanking region and exon 1 of the Kell gene.
Figure 4 shows an autoradiogram illustrating the promoter activity of the 5' flanking region of the Kell gene.
Figure 5 shows a comparison of the 3' ends of the Kell gene obtained from various clones of the gene.
Figure 6 shows an ethidium bromide/agarose gel illustrating a differential separation of amplified DNA corresponding to specific fragments of the Kell gene.
Figure 7 shows a comparison of corresponding portions of K1 DNA and K2 DNA.
Figure 8 shows an ethidium bromide/agarose gel illustrating a differential separation of amplified DNA corresponding to K1 DNA and K2 DNA according to the method of the invention (not according to the invention).
Figure 9 shows an ethidium bromide/agarose gel electrophoresis resolution of PCR products obtained according to the method of the invention.
Figure 10 shows a comparison of corresponding portions of K6 DNA and K7 DNA.
Figure 11 shows an ethidium bromide/agarose gel electrophoresis resolution of K6/K7 PCR products obtained according to the method of the invention.
Figure 12 shows an ethidium bromide/agarose gel electrophoresis resolution of K6/K7 PCR products obtained according to the method of the invention.
Figure 13 shows a comparison of corrsponding portions of K10 DNA and K(-10) DNA.
Figure 14 shows a comparison of corresponding portions of K3 DNA, K4 DNA, and K21 DNA.

### DETAILED DESCRIPTION OF THE INVENTION

The Kell blood group system is distinguished by its antigenic complexity (Ref. 18). Over 20 different antigens have been ascribed to the Kell system. A majority of the antigens are organized in 5 antithetical sets of high and low frequency antigens, with other Kell antigens being independently expressed. The molecular basis for this antigenic diversity has not previously been understood. The present invention, which involves description of the organization of the KEL gene and the boundaries of its 19 exons, now enables the molecular characterization of the different Kell phenotypes.

The molecular basis of the different Kell phenotypes has not previously been determined. Having now studied the structure of the KEL gene, and identified for the first time the 19 exons which encode Kell protein, molecular bases of polymorphisms in the Kell genotype have now been determined.

In particular, the K1/K2 polymorphism and its locus has now been determined by sequencing the exons of K1/K1 DNA and comparing them to K2/K2 sequences. It was unexpectedly been found that base substitution in exon 6 of the X1 genotype predicts an amino acid change. This base substitution also creates a restriction enzyme site which has been employed to test over 40 different samples to confirm that the base substitution identifies the K1 genotype.

As another of the Kell group of antigens, K6 (also known as Js^{a}) is a low frequency red cell antigen which has an allelic relationship with the high frequency K7 antigen. K6 appears in less than 1.0% of the total population, but up to 19.5% of African-Americans carry the K6 antigen. We have now determined the molecular basis of polymorphism in the K6/K7 polymorphism by sequencing the 19 exons of the Kell gene (KEL) of a K:6,-7 person. Comparisons of the sequence obtained from K:6,-7 DNA with that of K:6,7 DNA showed an unexpected base substitution in exon 17 that predicts an amino acid change from leucine to proline. This base change also causes a deletion of a restriction enzyme cleavage site. Another base change in K7 is silent in that it does not result in change in the expressed protein, yet it causes the deletion of another restriction enzyme cleavage site which can be exploited to differentiate K6/K7 genotype.

The K10 (Ul^{a}) antigen is a low frequency Kell antigen for which there is no corresponding serologically detectable high frequency antigen. Thus, the wild-type phenotype (designated herein as K(-10) is characterized by a lack of the K10 antigen. It has now been determined that the molecular basis of the K10 polymorphism resides in exon 13. A point mutation at nt 1601 has been found to predict an amino acid change from glutamic acid to valine. This base change also creates a new restriction enzyme cleavage site which can be employed to determine K10 genotype according to the invention.

Another of the antithetical allelic sets in the Kell genotype is the K3/K4/K21 (Kp^{a}/Kp^{b}/Kp^{c}) set. Our study of the KEL gene has also unexpectedly revealed that the three alleles in this set may be differentiated on the basis of changes in two adjacent nucleotides in exon 8, each of which predicts a discrete amino acid change at the same position in the amino acid sequence of the Kell protein. Each of the base changes also creates a new and distinct restriction enzyme cleavage site in the gene.

The most prevalent Kell phenotype is K:-1, 2, -3, 4, -6, 7, 9, 11, 12, 14, 18, 19, 22. We have now defined the 19 exons of the KEL gene of a person with common Kell phenotype. To determine the molecular basis of Kell polymorphisms we designed a series of primers which would amplify the 19 exons of KELL. We then compared the DNA sequences of persons expressing various combinations of high and low frequency antigens to determine sequence variations potentially related to the observed variations in antigen expression.

For example, we compared the DNA sequences of K1/K1 and K2/K2 DNA. The only base change which would encode a different amino acid was found in exon 6, and changed a threonine to a methionine at a consensus N-glycosylation motif (Asn.X.Thr - Met). This change would prevent N-linked glycosylation at this site. Based on the amino acid sequence of Kell protein of common phenotype, there are 6 possible N-glycosylation sites, i.e., asparagine residues 93, 115, 191, 345, 627 and 724. However, the asparagine at position 724 is probably not glycosylated because it is part of a sequence Asn.Pro.Ser and the presence of proline between asparagine and serine/threonine inhibits N-glycosylation (Ref. 31). In any event, changing threonine to methionine at position 193 would prevent glycosylation at asparagine 191. Thus, K1 protein would be composed of at most 4 instead of 5 carbohydrate moieties. Lack of a carbohydrate side-chain may expose different parts of the protein leading to immunogenicity. The loss of glycosylation in a red cell surface protein can lead to a change in blood group phenotype. The Webb glycophorin C variant also lacks an N-glycan (Refs. 32-33).

It has also now been observed that the point mutation from C to T in exon 6 also creates a new restriction enzyme site, 5'-GAATGCT-3', which can be cut by BsmI, a well known restriction endonuclease. The use of restriction enzyme digestion allows the differentiation of K1/K1 and K2/K2 homozygotes and of K1/K2 heterozygotes, permitting the development of a diagnostic genotype procedure.

While not wishing to be bound by theory, it is known that, in very rare cases, red blood cells will be observed which do not express any Kell antigens. In this phenotype, known as Kₒ (null), the red cells appear not to have any Kell protein on the cell membrane. Yet, preliminary experiments in our laboratory indicate that two Rₒ persons contain Kell mRNA in peripheral blood and that the sequence of the mRNA from the initiation ATG codon to the poly A tail is identical to mRNA obtained from persons with common Kell phenotype. This indicates that the base sequences in the 19 exons of K2 and of some Kₒ persons are identical. Therefore, PCR amplification of exon 6 and genotyping by treatment with BsmI could indicate a K2 genotype in Kₒ persons. Serological analysis easily detects the Kₒ homozygote, but the Kₒ heterozygotes would be serologically identified as K2 or K1, and *Bsm*I analysis would provide no further discrimination. Presumably, the same would occur in the Kmod phenotypes in which expression of all Kell-related antigens are weakened. Nonetheless, for practical purposes, such ambiguous phenotypes are less clinically significant since Kₒ and Kmod phenotypes are very rare. Kₒ heterozygotes would be phenotypically, and therefore clinically, equivalent to K1 or K2 homozygotes. What is generally important in K1-sensitized pregnancies is to identify whether the fetus is a K1 carrier or not. The method of the invention now allows identification of Kell genotype, including identification of the K1 gene.

We have also now determined the molecular basis of polymorphism in the K6/K7 allelic set ("K6/K7 polymorphism") by sequencing the 19 exons of the Kell gene (KEL) of a K:6,-7 person. Comparisons of the sequence obtained from K:6,-7 DNA with that of K:6,7 DNA showed a T to C base substitution at nt 1910 in exon 17 that predicts an amino acid change from leucine in K7 to proline in K6. The T to C substitution at nt 1910 eliminated an *Mnl*I restriction enzyme site found in K7. Analysis of a 111 bp PCR-amplified product of exon 17 spanning the T to C substitution was performed with *Mnl*I. Eight unrelated persons with the K6 phenotype were tested and all contained the T to C substitution at nt 1910. Another base substitution from A to G at nt 2019 in exon 17 was also noted, but this point mutation did not encode a different amino acid. Even so, this substitution was found to create a new *Dde*I restriction enzyme site. Thus, these base substitutions permit diagnostic procedures for the differentiation of K6 from K7 according to the invention.

The molecular bases of the K3/K4/K21 polymorphism have also now been characterized. Two discrete changes in a single codon have been found to be associated with changes in protein expression from K4 to either of K3 or K21. The same changes also encode new and unique restriction cleavage sites which specifically characterize K3 and K21 nucleic acid as distinct from wild-type K4 nucleic acid. In the case of K3, a C 961 T substitution results in an Arg 281 Gln change. This base substitution also creates a new *Nla*III restriction enzyme site. In the case of K21, a G 962 A substitution encodes Arg 281 Gln. This substitution also creates a new PvuII restriction enzyme site. Accordingly, the differentiation of K4 from K3 and K21 genotypes is possible by employing the diagnostic method of the invention.

For purposes of more clearly and accurately describing the invention herein, certain terminological conventions have been adopted in the following discussion. These conventions are intended to provide a practical means for enhancing description of the invention, but are not intended to be limiting, and the skilled artisan will appreciate that other and additional interpretations may be implied.

As used herein, the term "restriction fragment length polymorphism" or "RFLP" refers to the differences in DNA nucleotide sequences that are randomly distributed throughout the entire genome and that produce different restriction endonuclease patterns for different individuals upon digestion of genomic DNA. Likewise, "restriction site" refers to a site in a nucleic acid at which a restriction enzyme cleaves the nucleic acid. Cleavage of a nucleic acid by a restriction enzyme generates pieces of nucleic acid termed "restriction fragments". When a restriction site is gained or lost as a result of mutation or allelism, the restriction fragment pattern can vary, yielding information about the location of the site.

"Polymorphic" or "DNA polymorphism" refers to the condition in which two or more variations of a specific DNA sequence coexist in the same interbreeding population. Polymorphic differences are caused by differences in amino acid sequence which may be due to point mutations, gene rearrangements or alternative splicing. "Alleles" are genes which are polymorphic variants of one another and occur alternatively at a given locus in the genome.

A gene encoding a Kell protein is designated a "Kell gene". The region of a Kell gene which encodes a specific domain of a Kell protein is termed a "Kell locus". For example, it has been determined as a result of the present invention that the K1 antigen and the K2 antigen are encoded by alleles of the Kell gene. More specifically, the K1 antigen and the K2 antigen are alternate versions of the same domain of the Kell protein, in which a single amino acid substitution has been found to result in the difference between Kell protein including the K1 antigen and Kell protein including the K2 antigen. This single amino acid substitution has now been found to arise from a single nucleotide substitution in the Kell gene. Thus, the portion of the Kell gene which includes this site of nucleotide variation is designated the "K1/K2 locus". When the K1/K2 locus encodes K1 antigen this region of the Kell gene is designated the "K1 locus". When the K1/K2 locus encodes K2 antigen, this region of the Kell gene is designated the "K2 locus". The K1/K2 locus is also said to be the site or locus which determines or characterizes the "K1/K2 polymorphism", i.e., the observed phenotypic difference between the K1 and K2 antigens. Similarly, other Kell loci determine or characterize other Kell polymorphisms, such as the K6/K7, K10/KC-10), and K3/K4/K21 polymorphisms.

For purposes of this invention, Kell protein which includes a particular domain of interest may be designated according to that domain regardless of the other domains in the protein. Thus, if a Kell protein includes the domain responsible for K1 antigen, the protein may be designated "K1 protein", while Kell protein which includes the K2 antigen or K2 domain is designated "K2 protein". Using similar analysis, DNA encoding K1 protein may be termed "K1 DNA", while DNA encoding K2 protein may be termed "K2 DNA" regardless of whether or not the DNA further encodes other Kell domains. It is also to be understood that other Kell-related or Kell-based chromosomal DNA (including exons and introns and parts thereof), nucleic acid templates, nucleic acid transcripts, as well as cDNA, may be similarly designated as K1/K2, K1, or K2, depending on context or application. Similar considerations apply in the context of other antigens in the Kell system, such as the K6/K7, K10, and K3/K4/K21 antigens.

As a result of the present invention, it is now possible to design probes and/or primers comprising nucleic acid oligomers or oligonucleotides which can be employed to detect polymorphisms in the Kell gene. The probes or primers suitable for this purpose preferentially hybridize with or are specific for a region of the Kell gene comprising a site or region in which a point change in nucleotide sequence causes a change in the Kell gene product characterizing a polymorphism. Accordingly, the probes or primers include those which hybridize with higher frequency alleles as well as those which hybridize with lower frequency alleles. In certain applications, it is desirable to define the presence of heterozygosity. In such cases probe or primer combinations enabling differential detection of two or more alleles and/or loci have been found to be useful.

For example, a probe of the invention may be said to bind to or hybridize with Kell DNA if it specifically recognizes a defined region of Kell DNA. Greater precision is intended when a probe is said to hybridize with a specific allele such as a "K1 probe" which is understood to hybridize with K1 DNA.

A primer of the invention may be said to specifically amplify Kell DNA if it binds to or causes elongation through a defined region of Kell DNA. Thus, a primer specifically amplifies K1/K2 DNA if it preferentially amplifies a region including the K1/K2 locus. Accordingly, a K1/K2 primer amplifies DNA including the K1/K2 locus but non-selectively amplifies both K1 DNA and K2 DNA. On the other hand, a primer is specific for a particular allele, such as K1 (K1 primer), if it specifically amplifies only DNA associated with that allele, such as K1 DNA, while a primer specific for K2 (K2 primer) specifically amplifies only K2 DNA. Similar considerations apply to other polymorphisms in the Kell system.

It is particularly preferred that probes be capable of differentiating two alleles which differ by no more than a single nucleotide modification. It is known in the art that it is possible to control the specificity of hybridization by selectively constructing probes and primers and by adjusting hybridization or other experimental conditions. There may be situations, however, in which it would be desirable to employ probes which hybridize somewhat less selectively. Accordingly, it is within a particular context that a probe or primer according to the invention is said to be "substantially complementary" to a specific Kell sequence. That is, if the situation demands high precision, a probe or primer is substantially complementary to a target sequence if there exists only a very small probability that the oligomer will bind with a sequence other than the specific target sequence. In other situations, a probe or primer may be deemed to be substantially complementary to a target sequence if the probability of a unique hybridization is substantially less than 1.0. Thus, a probe or primer of the invention may be "substantially complementary" to a target region if it is either exactly complementary, or even only partially complementary, to the target region, depending on the required stringency of the method parameters.

Thus, the invention provides probes and primers which hybridize with parts or all of the Kell locus. Such probes are useful when the Kell gene or transcripts thereof are desired to be characterized. Moreover, the invention provides probes and primers which include part or all of one or more introns in the Kell locus in chromosomal DNA. Such probes are useful when the Kell gene itself is desired to be characterized and additional information is desired to be obtained about the structure of the gene in a particular individual.

The probes or primers of the invention may also include, as part of their nucleotide sequences, regions which are not substantially complementary to any region adjacent to or near a target sequence. Thus, in any probe or primer of the invention, at least a part of the probe or primer is substantially complementary to a target segment.

"Amplification" refers to any molecular biology technique for detection of trace levels of a specific nucleic acid sequence by exponentially amplifying a template nucleic acid sequence. Techniques are known for the amplification of DNA as well as RNA, and for the generation of amplified RNA from DNA and vice versa. In particular, amplification techniques include such techniques as polymerase chain reaction (PCR), and ligase chain reaction (LCR). The PCR is known to be a highly sensitive technique, and is in wide use. The LCR is known to be highly specific, and is capable of detecting point mutations. In certain circumstances it is desirable to couple the two techniques to improve precision of detection. The PCR is a well-known technique and is described, for example, in Innis et al. (Ref. 34). The LCR is more recently developed and is described in Landegren et al. (Ref. 35) and Barany et al. (Ref. 36). An LCR kit is available from Stratagene. Other amplification techniques may be expected to be employed according to the method of the invention.

"Primer" refers to an oligonucleotide, whether natural, cloned, or synthetic, which is capable of initiating nucleic acid synthesis for exponential amplification of a target or template nucleic acid sequence by an amplification technique. For PCR the primer acts as a point of initiation of DNA synthesis under conditions in which synthesis of a primer extension product complementary to a nucleic acid strand is induced, i.e., in the presence of four different nucleoside triphosphates and an agent for polymerization (i.e., DNA polymerase or reverse transcriptase) in an appropriate buffer and at a suitable temperature. For LCR the primer is capable of annealing to a target nucleic acid and of being ligated to an adjacent primer to serve as a template for amplification. Also for purposes of the LCR, the primer generally includes paired sets of adjacent, complementary oligonucleotides which can anneal to single stranded target molecules and ligate them together. For LCR amplification of DNA, the primers include two sets of adjacent, complementary oligonucleotides.

A primer is preferably an oligodeoxyribonucleotide and is single stranded for maximum efficiency in amplification, but may also be double stranded. If double stranded, the primer is generally first treated to separate its strands before being used to prepare extension products. Typically. LCR primers are double stranded. The exact length of a primer will depend on many factors, but typically ranges from 15 to 25 nucleotides. Short primer molecules generally require cooler temperatures to form sufficiently stable hybrid complexes with the template. A primer need not reflect the exact sequence of the template but must be sufficiently complementary to hybridize with a template. An example of a non-complementary sequence which may be incorporated into the primer is a sequence which encodes a restriction enzyme recognition site (see U.S. Pat. No. 4,800,159).

"Primer", as used herein, may refer to more than one primer, particularly in the case where there is some ambiguity in the information regarding one or both ends of the target region to be amplified. For instance, if a nucleic acid sequence is inferred from a protein sequence, an operable "primer" may actually include a collection of primer oligonucleotides containing sequences representing some or all of the possible codon variations based on the degeneracy of the genetic code. One of the primer oligonucleotides in this collection will be homologous with the end of the target sequence. Likewise, if a "conserved" region shows significant levels of polymorphism in a population, mixtures of primers can be prepared that will amplify adjacent sequences.

A primer or probe according to the invention can be labeled, if desired, by incorporating a label detectable by spectroscopic, photochemical, biochemical, immunochemical, or chemical means. For example, useful labels include ³²P, fluorescent dyes, electron-dense reagents, reporter molecules such as enzymes (as commonly used in ELISAs), biotin, or haptens or proteins for which antisera or monoclonal antibodies are available. A label can also be used to "capture" the primer, so as to facilitate the immobilization of either the primer or amplified DNA on a solid support.

"Oligonucleotide" or "nucleic acid oligomer" refers to primers, probes, nucleic acid fragments to be detected, nucleic acid controls, and unlabeling blocking oligomers and is defined as a molecule comprised of two or more deoxyribonucleotides or ribonucleotides. The nucleic acid oligomers of the invention may be single-stranded oligomers of deoxyribonucleic acid (DNA) or ribonucleic acid (RNA). The exact size of an oligonucleotide will depend upon many factors and the ultimate function or use of the oligonucleotide. The oligodeoxyribonucleotides and oligoribonucleotides may be obtained or derived by known methods from natural sources. Alternatively, the oligonucleotides may be produced synthetically according to methods known in the art. Such methods include, for example, cloning and restriction of appropriate sequences and direct chemical synthesis by a method such as the phosphotriester method of Narang et al. (Ref. 37); the phosphodiester method of Brown et al. (Ref. 38); the diethylphosphoramidite method of Beaucage et al. (Ref. 39); and the solid support method in U.S. Patent No. 4,458,066. It is preferred that the oligomers be at least substantially purified to avoid introduction of artifacts into the genotype determination method.

The invention also provides oligonucleotides which are structurally homologous to a part or all of genetic material related to or derived from the Kell gene. An oligomer is said to be "substantially homologous" to another if, despite variations in the nucleotide sequence, the oligomer encodes an amino acid sequence which is phenotypically identical to the sequence to which it is being compared. Thus, sequences are not substantially homologous if, when expressed, the sequences result in shift in Kell phenotype. On the other hand, two sequences encoding the same locus or sublocus are substantially homologous if they are not identical but nonetheless encode sequences which correspond to parts or all of phenotypically non-differentiable variants of the Kell protein. Accordingly, sequences which encode K₁ domain and sequences which encode the K2 domain are not substantially homologous, even though they differ, as has now been discovered, by a single nucleotide substitution. In contrast, variations in nucleotide sequence which do not result in an amino acid shift in the encoded gene product may be substantially homologous.

The method of the invention is a molecular genetic method which permits the determination of one or more aspects of the Kell genotype of a subject. The method generally involves obtaining DNA or other nucleic acid from a biological sample from a human subject or patient. Typically, the method requires a blood sample, but other types of tissue samples which contain erythroid tissue are useful. The method may be employed to test any individual for Kell genotype. In a particularly preferred embodiment, however, the invention provides a method for determining Kell blood group genotype in a fetus. In this embodiment, the preferred tissue sample includes a sample of amniotic fluid. It is understood, however, that such samples can be obtained from sample libraries or tissue archives such as blood banks, or from forensic evidence, etc. Accordingly, the method may be used on a unique or irreplaceable sample or may be used to screen large numbers of samples.

In general, the method of the invention includes generating at least one characteristic nucleic acid product from a nucleic acid sample. The sample nucleic acid is obtained or derived from a subject for whom Kell genotype is desired to be determined, and preferably includes a nucleotide sequence which contains a Kell polymorphism locus, although the exact sequence or structure need not be well defined beforehand. In preferred embodiments the nucleic acid sample includes genomic DNA of the subject.

The method or the invention generates nucleic acid product, characteristic of specific Kell genotype, in an amount sufficient to permit characterization of at least one aspect of Kell genotype. The method of the invention generates characteristic nucleic acid product which permits the differentiation of alleles of K6 and K7 Kell polymorphism.

In one preferred embodiment, the diagnostic method of the invention includes exposing Kell DNA to a restriction enzyme which differentially cleaves or digests nucleic acid containing a Kell polymorphism locus. Typically, the restriction enzyme generates a nucleic acid product which includes at least one restriction fragment which is distinctive in size and can be associated with a specific Kell allele on a consistent basis. For example, the restriction enzyme may digest one allele of a polymorphism set while failing to digest other alleles of the set. Alternatively, the restriction enzyme cleaves alleles in the set except for one which is left intact and is recognizable on that account. Numerous restriction enzymes are known in the art, and any restriction enzyme which produces at least one distinctive or characteristic fragment in this way may be employed according to this embodiment of the method of the invention.

For K1/K2 differentiation, a preferred restriction enzyme is BsmI which cuts at a restriction site which, it has now unexpectedly been found, occurs in K1 DNA but not in K2 DNA. Thus, *Bsm*I cleaves K1 DNA while leaving K2 DNA intact. For the K6/K7 polymorphism, preferred restriction enzymes include *Mnl*I and DdeI, which unexpectedly cleave K7 DNA but not K6 DNA. For K10, a preferred restriction enzyme is AccI, which unexpectedly cleaves K10 DNA, but not wild-type K(-10) DNA at that locus. In the K3/K4/K21 group, preferred restriction enzymes include *Nla*III, which unexpectedly cleaves only K3 (Kp^{a}) DNA, and/or PvuII, which unexpectedly cleaves only K21 (Kp^{c}) DNA; K4 (Kp^{b}) DNA remains uncleaved by either of these enzymes. Combinations of these or other enzymes may be employed as desired.

In another preferred embodiment, the diagnostic method of the invention involves differentially amplifying DNA including a Kell polymorphism locus. Preferably, the Kell DNA is amplified by means of a primer which differentially amplifies a specific allele in a set of alleles associated with a Kell polymorphism. For example, by carefully selecting an appropriate amplification primer, DNA containing a K1 locus may be amplified to produce a distinctive amplified product, while DNA containing a K2 locus is not amplified to any substantial degree. Alternatively, a set of primers may be employed which produce distinctive set of amplified products which may be analyzed to determine the presence or absence of alleles. For example, a K1 specific primer may produce a product characteristic of K1 genotype and a K2 primer may produce a product characteristic of the K2 genotype. Other alleles of other Kell polymorphisms may also be differentially amplified by selecting appropriate primers.

Any combination of primers may be employed according to this embodiment provided that the amplification product(s) permits characterization of a desired aspect of Kell genotype. Preferred primer sets include: primers which differentially amplify K1 and K2 DNA; primers which differentially amplify K6 and K7 DNA; primers which differentially amplify K10 and K(-10) DNA; primers which differentially amplify K3, K4, and K21 DNA; or combinations thereof.

It is among the advantages of this approach that, by using appropriate primer combinations, specific fragments of DNA are amplified to provide products in an amount sufficient to characterize Kell genotype without any requirement for digesting the DNA with restriction enzymes. Nonetheless, it is within the invention to employ primers which provide differentiating sets of DNA fragments, and to supplement the differentiation of products and consequent characterization of Kell genotype by means of selectively cleaving the fragments with restriction enzymes. This may be particularly helpful in situations where some fragments are not completely separable or differentiable due to limitations of technology, while selective cleavage of the fragments resolves an ambiguity.

The diagnostic methods of the invention typically yield a nucleic acid product, such as digested DNA or RNA fragments or amplified DNA or RNA products, in amounts sufficient to permit the characterization of Kell genotype. The pieces of nucleic acid permit such characterization since they carry information in their sequences and/or sizes which permits at least some of the fragments to be differentiated from the others. The differentiating characteristics of the fragments may be exploited by any methods known in the art.

For example, fragments may be separated from each other on the basis of physical characteristics. A highly preferred differentiating characteristic is molecular weight. Specific and characteristic molecular weights of the fragments are directly determined by site-specific cleavage by restriction enzymes. Particular primers may generate amplification products having specific molecular weights. The fragments may be separated from one another by methods such as polyacrylamide gel electrophoresis which relies on molecular weight as well as net molecular charge for separation. Such techniques produce informative patterns of fragments permitting determination of Kell genotype.

Kell nucleic acid products may also be differentiated from one another on the basis of their sequence identity. Specific probes may be employed according to known methods to hybridize specifically or uniquely with specific fragments. By the differential labeling of several probes, the presence or absence of particular Kell polymorphism-specific fragments may be established. Alternatively, following separation of fragments, their detection may be enabled by marking or staining some or all of the fragments specifically or non-specifically according to known methods. Various sequence non-specific marker substances are known, such as ethidium bromide.

Hybridization probes may be employed to detect the presence or absence of specific Kell nucleic acids in a testable system. Diagnostic tests based on hybridization techniques can be both extremely specific and highly sensitive, providing information characterizing Kell genotype. Such techniques are described, for example, in Glick BR and Pasternak JJ, Molecular Biotechnology: Principles and Applications of Recombinant DNA, ASM Press, Washington, D.C., pp. 192-201 (1994) (Ref. 40).

In a preferred embodiment, the diagnostic method includes:
(a) obtaining DNA from a tissue sample from a patient;
(b) amplifying the DNA by polymerase chain reaction using a primer or primer set which specifically amplifies DNA characterizing or determining a specific Kell polymorphism;
(c) selectively cleaving the amplified DNA by a restriction enzyme which differentially cleaves Kell DNA to produce a pattern of DNA fragments; and
(d) separating the DNA fragments according to molecular weight to form a pattern of DNA fragments,
wherein the pattern of DNA fragments provides specific information characterizing the Kell genotype of the patient with regard to the specific Kell polymorphism.

In another preferred embodiment, the diagnostic method includes:
(a) obtaining DNA from a tissue sample from a patient;
(b) amplifying the DNA by polymerase chain reaction using a primer or primer set comprising at least one primer which amplifies only DNA of one allele in a Kell allelic set; and
(c) separating the amplified DNA according to molecular weight to form a pattern of DNA fragments,
wherein the pattern of DNA fragments provides specific information characterizing the genotype of the patient with respect to the specific Kell polymorphism.

In still another embodiment, the diagnostic method includes:
(a) obtaining DNA from a tissue sample from a patient;
(b) amplifying the DNA by ligase chain reaction using a primer set which amplifies only DNA of a Kell allelic set to produce a pattern of DNA fragments; and
(c) separating the DNA fragments according to molecular weight to form a pattern of DNA fragments,
wherein the pattern of DNA fragments provides specific information characterizing the genotype of the patient with respect to the Kell polymorphism.

Moreover, in another embodiment, the diagnostic method includes:
(a) obtaining DNA from a tissue sample from a patient;
(b) selectively cleaving the DNA by a restriction enzyme which differentially cleaves DNA of at least one allele in a Kell allelic set to produce a pattern of DNA fragments;
(c) amplifying the pattern of DNA fragments by polymerase chain reaction using a primer or primer set which amplifies only DNA of the allelic set; and
(d) separating the amplified DNA according to molecular weight to form a pattern of DNA fragments,
wherein the pattern of DNA fragments provides specific information characterizing the genotype of the patient with respect to the Kell polymorphism.

In yet another embodiment, the invention (not according to the invention) provides a diagnostic method which includes:
(a) obtaining DNA from a tissue sample from a patient;
(b) exposing the DNA to a restriction enzyme which differentially cleaves K1 DNA and K2 DNA to produce a pattern of DNA fragments;
(c) separating the DNA fragments according to molecular weight to form a pattern of DNA fragments; and
(d) determining the presence of K1 DNA and/or K2 DNA using detectably labeled Kell cDNA probes;
wherein the pattern of DNA fragments provides specific information characterizing the K1/K2 genotype of the patient. Such methods include, for examples, Southern blot methods such as are known in the art.

In an alternative embodiment, the invention (not according to the invention) provides a diagnostic method for detecting a target nucleic acid which is specific to K1 or K2. In this embodiment, the method includes:
(a) obtaining a nucleic acid fraction from a tissue sample from a patient;
(b) ascertaining the presence in the nucleic acid fraction of a target nucleic acid encoding at least a part of the Kell protein and including the site characterizing the K1/K2 polymorphism. In this method, the presence of the target nucleic acid is ascertained by means of a probe nucleic acid which includes a nucleic acid sequence which is known to specifically bind to or hybridize with K1 DNA or K₂ DNA or transcripts thereof. The probe nucleic acids useful in this method may be any of the Kell-based oligomers described herein, including those which are detectably labeled or attached to a substrate. This method may be used to detect target nucleic acids which are chromosomal or genomic DNA, mRNA, and cDNA, as well as other related forms of nucleic acid encoding the K1/K2 domain of the Kell protein. Such methods include, for example, dot blot methods such as are known in the art.

In still another embodiment, the invention provides a method for the determination of Kell blood group genotype. In this embodiment, the method includes the steps of:
(a) selecting a probe nucleic acid sequence substantially corresponding to at least part of a known Kell exon or a transcript thereof, wherein the known Kell exon comprises a Kell polymorphism locus and codes for a specific Kell allele;
(b) contacting a sample nucleic acid sequence obtained or derived from a subject, the subject having an unknown phenotype with respect to the Kell polymorphism locus, with the probe nucleic acid sequence under conditions which permit hybridization when the nucleic acid sequences are significantly complementary; and
(c) measuring an amount of hybridization between the probe nucleic acid sequence and the sample nucleic acid sequence.

In this embodiment, the detection of an amount of hybridization corresponding to an amount resulting from significantly complementary sequences indicates that the subject possesses the specific Kell allele under investigation. On the other hand, detection of an abnormally low amount of hybridization indicates that the subject lacks the specific Kell allele being investigated.

The invention further provides expression recombinant vectors which carry Kell nucleic acid sequences. Such vectors permit transformation of cells, particularly eukaryotic cells such as yeast and human cells, to cause such cells to express a heterologous protein product. The invention provides expression vectors which include a nucleic acid sequence which encodes at least a part of the Kell protein including: a part of the protein which encodes a site of Kell polymorphism. In particular, the invention provides expression vectors which carry K1 DNA permitting transformation of cells to produce transformed cells or transformants which express K1 protein on their cell surface. Vectors are also provided which carry K6 cDNA, as well as vectors which carry K10 cDNA, and vectors which carry K3 or K21 cDNA, permitting transformation of cells to express K6, as well as K10, and K₃ and K21, respectively. The invention also provides stable cell lines which have been modified (i.e., transformed) to express protein on its cell surface, as well as a method for transforming a cell line to express such protein. With respect to the K1 expression, such protein preferably includes at least the K1 domain, and more preferably, the protein is K1 protein. Similarly, with respect to expression of K6, K10, as well as K3 and K21, the expressed protein preferably includes the K6, the K10, the K3 or the K21, domain, respectively; more preferably, the protein is K6, K10, K3, or K21 protein, respectively. Methods for preparing expression recombinant vectors, and for transforming cell lines using such vectors are known in the art, and are described generally in Glick BR, and Pasternak JJ, Molecular Biotechnology; Principles and Applications of Recombinant DNA ASM Press, Washington, D.C. (1994) Chapter 5 (Ref. 40).

The Kell genotype determination method of the invention is of particular utility in the determination of the fetal Kell genotype. The molecular genetic method described herein can easily be applied to DNA samples obtained from amniotic fetal cells and is useful in determining the Kell genotype of the fetus. The methods of the invention are also useful in a variety of other situations in which molecular genetic information about a person is desired. For example, the methods of the invention are useful in situations in which it is desired to obtain information concerning the identity of an individual from forensic samples. Alternatively, the methods of the invention are useful for obtaining genetic information enabling the determination of paternity in those situations in which paternity is in doubt or dispute. In addition, the methods of the invention are useful for the determining the Kell genotype of a recipient of a blood transfusion, as well as for the screening of stored blood for Kell genotype, to avoid transfusion incompatibility. The peptide based methods of the invention are particularly useful in determining alloimmunization of a person to a Kell antigen, such as K1. Information about such alloimmunization would be expected to be useful in advising women about risk entailed in future pregnancies. Other applications of the methods of the invention will suggest themselves to the skilled artisan.

In addition, the genotype characterization method of the invention may be performed selectively so as to permit description of some or all of the Kell family of alleles. The method may also be performed in conjunction with the determination of phenotype of some or all of the Kell family of antigens. Thus, it is possible to confirm Kell genotype by employing serological testing in conjunction with genomic analysis using a restriction enzyme. Such serological testing steps may be performed either prior to, in parallel with, or after the prescribed genomic testing. Moreover, the planning of testing procedures may be based on preliminary partial results which rule out or imply the possibility of, for example, certain rare genotypes. It may also be desirable to perform genotype testing for other markers or blood groups together with the Rh genotyping method of the invention. For example, the genotyping method described in U.S. application Serial No. 08/ filed , 1995, (Attorney Docket No. 454-5) relating to the determination of Rh genotype, may be performed in conjunction with the method of the invention to provide counsel as to a broader spectrum of possible difficulties in pregnancy. In general, the diagnostic method, compositions and kits of the invention are expected to be employed in conjunction with a wide variety of known medical tests and assays to enhance and complement the information available to the clinician concerning a patient. See e.g., Walker RH et al., eds., Technical Manual, 10th ed., American Association of Blood Banks, Arlington, Virginia (1990) (Ref. 41).

The following examples are intended to assist in a further understanding of the invention. The particular materials and conditions employed are intended to be further illustrative of the invention and are not limiting upon the reasonable scope thereof.

For each of the examples described herein, the molecular biology techniques employed were performed generally in accordance with methods accepted in the art. see, for example, Sambrook et al. (Ref. 42), and Innis et al. (Ref. 34).

### Example 1

The organization of the KEL gene was determined using the following procedure.

### Screening of a Genomic Library

A human placental genomic DNA library constructed in EMBL3 Sp6/T7 was obtained from clontech, Inc. (Palo Alto, CA). The genomic DNA library was constructed by partially digesting human placenta genomic DNA with Sau3A1 and ligating the fragments into the DAMHI site of the vector. For screening, the X phage library was grown in either NM528 or LE392 strain of E. coli and plated. The DNA was lifted on Hybond"-N+ membranes (Amersham Co., Arlington Heights, IL) and hybridized with Kell cDNA probes according to standard procedures (Ref. 43). The cDNA probes were labeled with ³²P by random primer extension using a commercial kit (Boehringer Mannheim Inc., Indianapolis, IN). The specific activity of the probes was approximately 1 x 10⁸ cpm/µg. Fourteen positive clones were isolated.

### Characterization of Exons and Introns

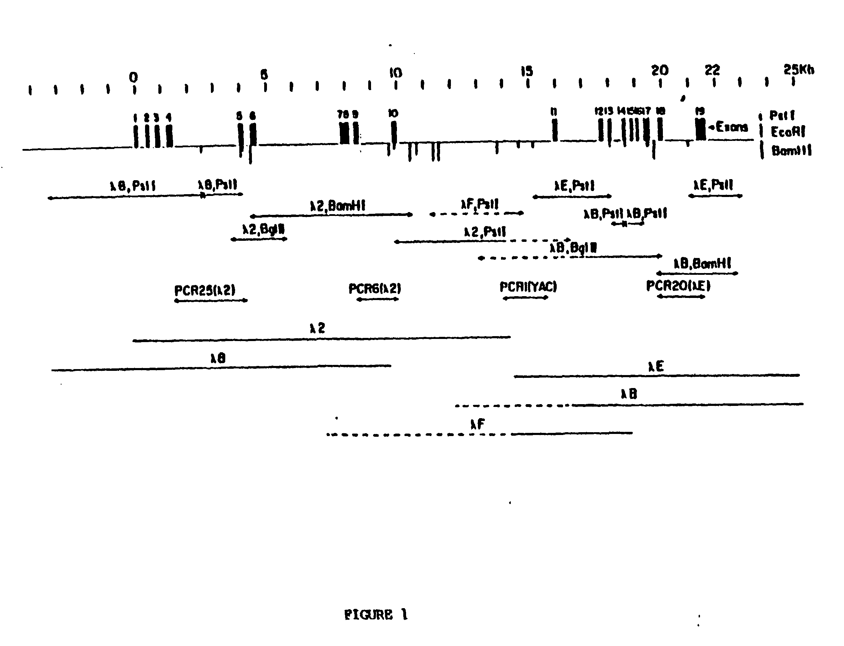

Of the 14 positive clones identified, five (λ2, λ8, λB, λF, and λE) were selected for further characterization. Two of the clones (λ2 and λE) span most of the Kell gene and cover about 21.5 Kb (see Figure 1). Clone λ8 was studied because it extended more of the 5' flanking region and also served to reconfirm the sequences determined from clone λ2. Clones λB and λF were used because they overlapped with λE, and in addition a small segment of λF overlapped with the 3' end of λ2 (see Figure 1).

These five clones were initially mapped by digestion with *Xho*I plus *Pst*I and with XhoI plus EcoRI, followed by Southern blot hybridization using different oligonucleotides specific for various locations in the Kell cDNA sequence. The genomic clones were then digested with the following enzymes: PstI, EcoRI, BamHI, BglII and XhoI, either individually or in combination. The individual gene fragments were subcloned into PUC18 (Gibco BRL, Gaithersburg, MD) or pGEM-3Zf(+) (Promega Co.) and sequenced on an automated 373A DNA sequencer (Applied Biosystems, Inc., Foster City, CA). The exons, determined in relation to the Kell cDNA sequence, and their flanking regions, were fully sequenced, as were some short introns. With one exception, the long introns were sized by PCR using primers from the flanking regions of the introns. The longest intron, between exons 10 and 11, was partially sized using primers from known intron sequences obtained from λ2 and XE, using a YAC clone as template. (Seven yeast artificial chromosome (YAC) clones were separately isolated by PCR specific for KEL exon 6 from a YAC resource enriched for chromosome 7 DNA.) This area of the intron was confirmed by PCR using human genomic DNA as template.

### Restriction Enzyme Mapping and Southern Blot Analysis of the Genomic Clones

Subclones were mapped with BamHI, EcoRI and PstI. The different sizes of digested DNA were resolved by agarose gel electrophoresis. The DNA fragments were in some cases further analyzed by Southern blots using ³²P-labeled oligonucleotides derived from different locations of Kell cDNA and also identified with available sequences of the subcloned gene fragments. The combination of these procedures allowed a restriction map of BamHI, EcoRI and PstI to be constructed. Sequence analysis of the subcloned Kell gene fragments from these 5 genomic clones revealed that the human KEL gene contains 19 exons ranging in size from 63 bp (exon 7) to 288 bp (exon 19) which includes a 3' untranslated region.

Figure 1 shows the newly determined structure of the human Kell gene *(KEL)* with restriction enzyme sites. The position of exons are shown as dark boxes and the introns are shown as lines connecting the exons. The vertical lines of different heights mark the restriction enzyme sites in the Kell gene. The X phase, genomic clones are shown at the bottom of the diagram (λ2, λ8, λE, λB and λF). Two of the genomic clones from the commercial genomic library, clones λB and λF, gave inconsistent sequences when compared to other clones (λ2 and λ8). The 5' segments of clones λB and λF should be identical to the 3' segments of λ2 and λ8 but sequence analysis showed that they were different. The reason for this is not known, but could be due to ligation of DNA material during the preparation of the genomic library. These areas of difference are depicted in Figure 1 by broken lines. The restriction enzyme digested products which were subcloned and analyzed are shown above the genomic clones. Each sub-clone shows the restriction enzymes used and the genomic clones from which it was derived. Four PCR-derived sub-clones (PCR25, PCR6, PCR1 and PCR20) are also shown. In parenthesis each PCR product shows the genomic clone from which it was amplified. A small segment of the large intron not covered by clones λ2, λ8 and λE was determined by sequencing PCR products (PCR6) from a YAC clone (yWSS679) and from the genomic DNA. This strategy was employed even though the λF clone covers the 3' end of the λ2 clone. This allowed us to cover the entire Kell gene and to size the intron between exons 10 and 11 without ambiguity.

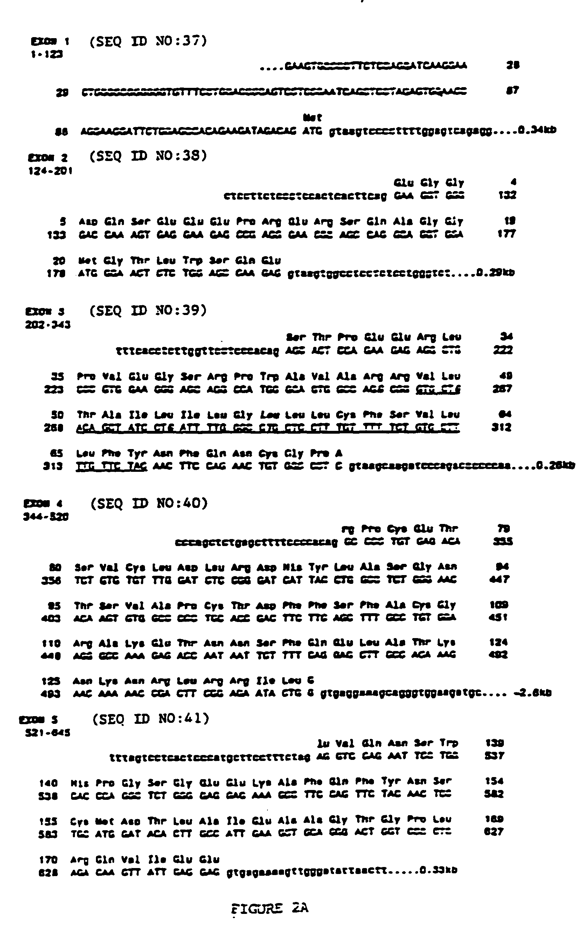

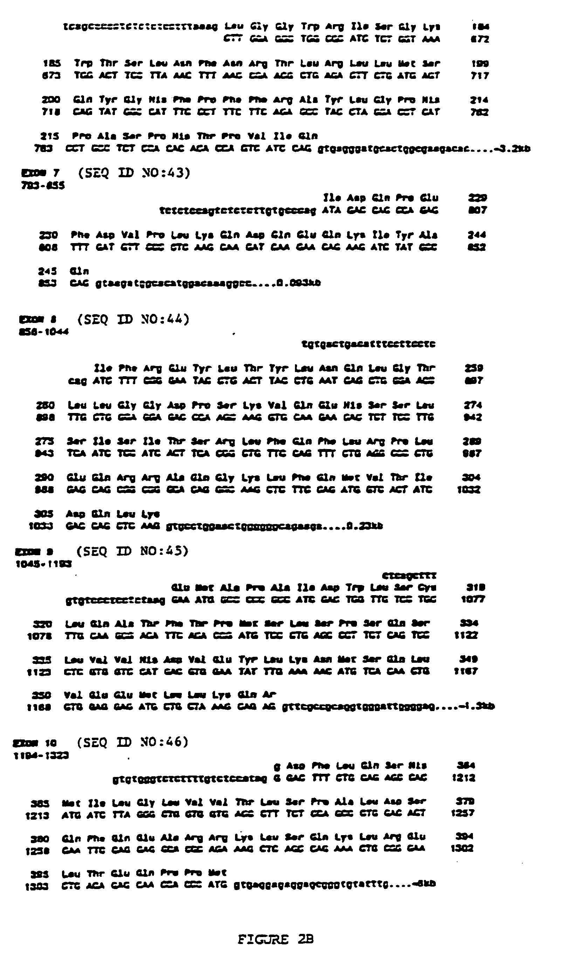

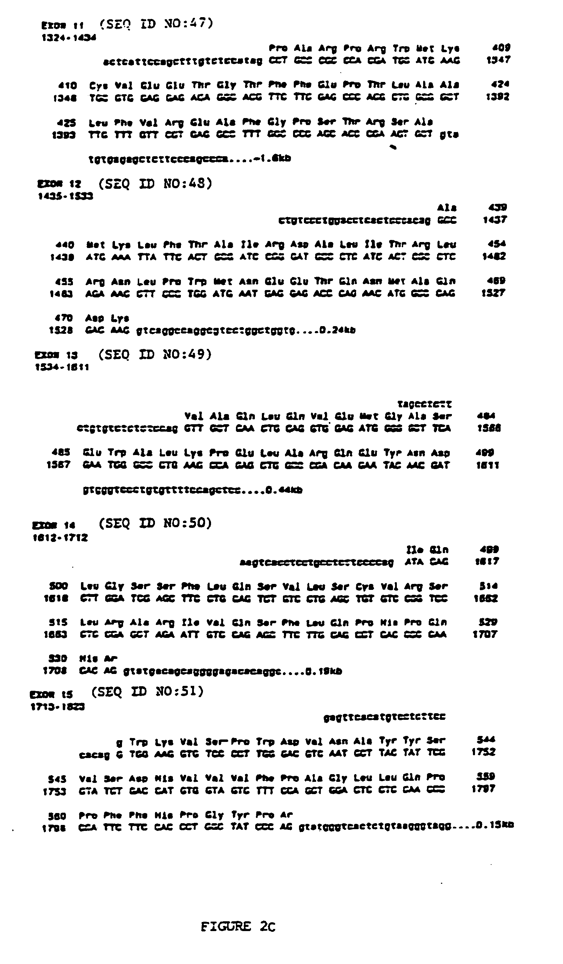

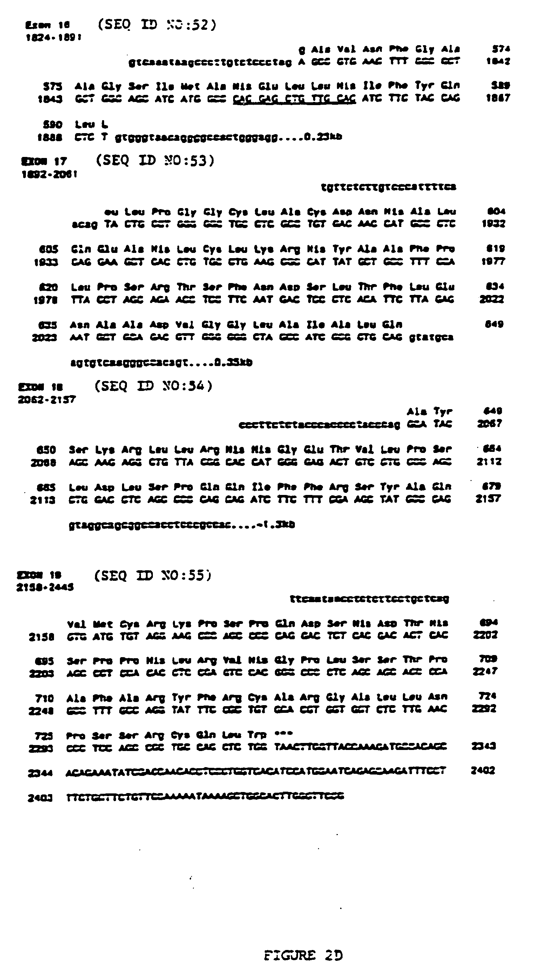

All exon/intron splice junctions were found to contain the 5' donor -gt and the 3' acceptor -ag sequences. The introns ranged in size from 93 bp to approximately 6 kb. there were 6 introns which were longer than 1 kb (Figs. 1 and 2). The long introns were not fully sequenced, but were sized by PCR. There was ambiguity in analyzing the intron between exons 10 and 11 because the 5' region of clones λB and λF, which were expected to overlap with the 3' region of clones λ2 and λ8, did not. These ambiguous areas of clones λB and λF are shown as dotted lines in Figure 1. Because of this uncertainty, the small gap of the gene not covered by clones λ2 and λE was bridged by PCR amplification of YAC clone yWSS679 containing the Kell gene (Figure 1). The size of this PCR amplified region was further confirmed by PCR of genomic DNA obtained from a person of common Kell phenotype using the same primers used for the YAC clone.

All of the exons were sequenced and compared to that of a full length Kell cDNA isolated from a human bone marrow library obtained from Clontech Laboratories, Inc. The Kell phenotype of this library was unknown. Differences were noted in 4 bases in exon 3 as compared with the published sequence for Kell cDNA (Ref. 14). These differences were due to sequencing errors in the original study. The corrected sequences were submitted to EMBL/GenBank Updates (National Center for Biotechnology Information, Bethesda, MD) under accession No. M64934. One notable difference is a base substitution in the membrane-spanning region, which encodes a leucine instead of proline. The corrected base sequences are shown in bold type in Figure 2.

Figures 2A-2D illustrate the sequence of the individual exons encoding human Kell protein with the immediate intron flanking splice junction sequences. The base sequences of the individual exons are shown in capital letters and the flanking intron sequences in small letters. The amino acid sequences encoded by the exons are shown above the base sequences. The numbers on the left-side, below the indicated exons, refer to the base numbers from the cDNA. The other numbers on the left and right sides indicate the amino acid residues and the bases, as previously described for the cDNA (Ref. 25). Intron sizes are specified at the end of the exons. 5' and 3' splice sites are also shown.

Since the Kell phenotype of the person from whom the genomic DNA library was constructed is unknown, we isolated RNA from peripheral blood of a person of known common phenotype (K:-1,2,-3,4,-6,7). cDNA was prepared by RNA-PCR and sequenced according to methods known in the art. The deduced amino acid sequence was identical to that shown in Figure 2 as obtained from the Clontech genomic library. In the person of known phenotype, C to T base differences occurred in two locations (nt 1656 and 1664), but these substitutions did not change the predicted amino acids.

Of interest is that exon 1 includes the 5' untranslated region and codes for only initiation methionine. The single membrane spanning region is located in exon 3 and the pentameric sequence (HELLH) which conforms with a consensus sequence (HEXXH) found in the active sites of zinc neutral endopeptidases (Ref. 43) is in exon 16 (Figure 2).

The coding region of the native protein is present in 18 of the 19 exons; the first exon contains the 5' untranslated region and the initiation methionine. Other examples having only the initiation codon in the first exon are known (Ref. 44). The single transmembrane region is encoded in exon 3 with exons 4 to 19 encoding most of the extracellular portion. The HEXXH motif, which is unique to zinc metallopeptidases (Ref. 29) is encoded in a 68 bp exon (exon 16). In addition to the consensus pentameric sequence, Kell has sequence and structural homology with a family of membrane-associated zinc neutral endopeptidases (EC24.11) (Ref. 25) of which the enkephalinases and CALLA are examples (Refs. 45-46). The CALLA gene is larger than the Kell gene, about 80kb, and is composed of 24 exons (Ref. 47). In CALLA the putative enzyme active site is encoded in exon 19 and a comparison of base sequences of exons 18 and 19 of CALLA with exons 15 and 16 of Kell shows 54.5% base identity.

### Example 2

### Transcription Initiation Site

The transcription initiation site of the *KEL* gene was determined by the rapid amplification of cDNA ends (5' RACE) employing a human fetal library (5' RACE Ready" cDNA library, Clontech laboratories, Palo Alto, CA). The source of poly (A)⁺ RNA was normal liver pooled from 2 female Caucasian fetuses, 22 and 26 weeks of gestation. The single stranded cDNA library was made by reverse transcription, using random hexamers as primers. An oligonucleotide anchor 3'-NH₃-GGA GAC TTC CAA GGT CTT AGC TAT CAC TTA AGC AC-p-5' (SEQ ID NO:6) was ligated to the cDNA. For the 5' RACE, 2 sets of nested PCR reactions were carried out, using primers from 2 different locations on Kell cDNA (nt 132 and nt 178). In the first PCR, 5' RACE Ready™ cDNA was used as template. The primers used for one set (PCR1) included the anchor primer provided by Clontech (5'-CTG GTT CGG CCC ACC TCT GAA GGT TCC AGA ATC GAT AG-3') (SEQ ID NO:7) and Kell anti-sense primer (5'-CTC GGC TCT TCC TCA CTT TGG TCC-3', nt 132) (SEQ ID NO:8). For the other set (PCR2), the primers included the Clontech anchor primer (SEQ ID NO:7) and Kell anti-sense primer (5'-CTC TTG GCT CCA GAG AGT TCC CAT-3', nt 178) (SEQ ID NO:9). For the second nested PCR the products of the first PCR reactions were used as template and the Clontech anchor primer (SEQ ID NO:7) was again used as sense primer for both parallel reactions. In PCR1 a Kell anti-sense primer (5'-CCC ACC TTC CAT CTG TCT ATC TTC-3', nt 109) (SEQ ID NO:10) was used. For PCR2, the Kell anti-sense, nt 132, (SEQ ID NO:8) was used.

PCR was performed in an automated thermocycler (Minicycler, MJ Research Inc. Watertown, MA) using an initial cycle of 94°C for 3 minutes, 62°C for 1 minute, and 72°C for 30 seconds. In cycles 2 to 30 the conditions were 94°C for 30 seconds, 62°C for 30 seconds and 72°C for 30 seconds. In the last cycle the polymerization step at 72°C was extended to 10 minutes. The final concentrations of reagents were 50 mM KCl, 10 mM Tris-HCl (pH 9.0), 3 mM MgCl₂, 400 nM of each primer, 200 µM of each dNTP, 0.1% Triton-X100, and 2.5 units of *taq* polymerase, in a final volume of 50 µL. The "hot start" method using Ampliwax™ from Perkin Elmer (Branchburg, NJ) was employed. The PCR products were separated by electrophoresis in 0.8% low melting agarose gels, eluted and directly ligated to pT7-Blue(R) plasmid vector from Novagen (Madison, WI) and transformed in DH5αF' strain of *E*. *coli.*

Each primer yielded 3 different size products. These products were subcloned and sequenced. The largest product from each PCR reaction had a 5' end at 120 bp upstream from the initiation codon. The 2 shorter PCR products ended at 81 and 30 bp upstream from the initiation codon.

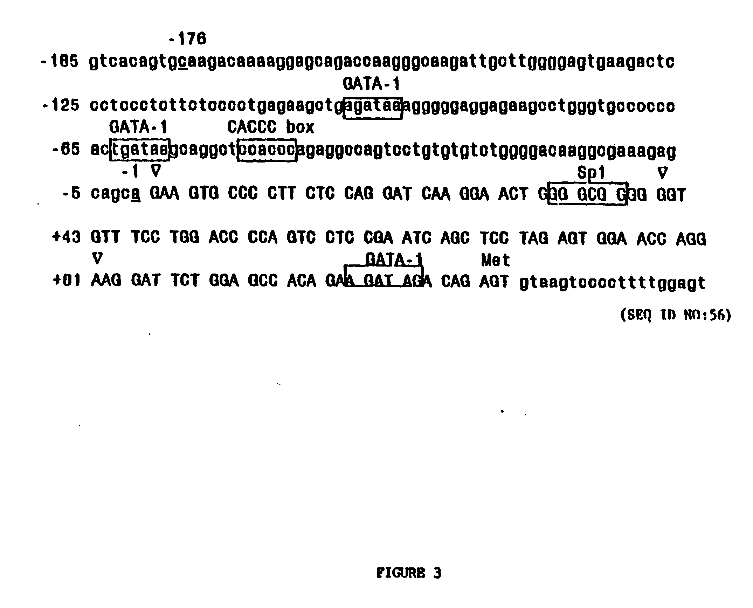

Figure 3 illustrates the nucleotide sequence of the KEL 5' flanking region showing exon 1 and possible cis regulatory elements. A 185 bp region upstream from the probable cap site is shown. The three possible transcription initiation sites are marked by V. Consensus sequences for GATA-1, Sp1 and a CACCC region are boxed. The region -176 to -1, which was copied by PCR and placed in a CAT-expression vector is shown. The initiation methionine is in bold letters.

Three possible transcription initiation sites were found using the 5' RACE procedure and poly (A)⁺ RNA from fetal liver. The first of these cap sites, located 120 bp upstream from the initiation ATG, is also the 5' end of a cDNA cloned from a human bone cDNA library (Ref. 25) the other 2 sites are 81 and 30 bp upstream from the initiation codon. All 3 sites were obtained using 2 different Kell cDNA anti-sense primers. Although all 3 locations are purine bases and could act as transcription initiation sites. It is also possible that those at 81 and 30 bp upstream from the ATG are artifactual due to incomplete reverse transcription. This is unlikely given that random hexamers were used to prepare the cDNA library. However, secondary RNA structures can also cause premature termination of reverse transcription.

### Example 3

### Analysis of 5' Flanking Region

The constitution of the 5' flanking region was obtained by DNA sequencing of sub-clone λ8, following digestion with *Pst*I (see Figure 1). The 5' flanking region was determined to span nucleotides -176 to -1.

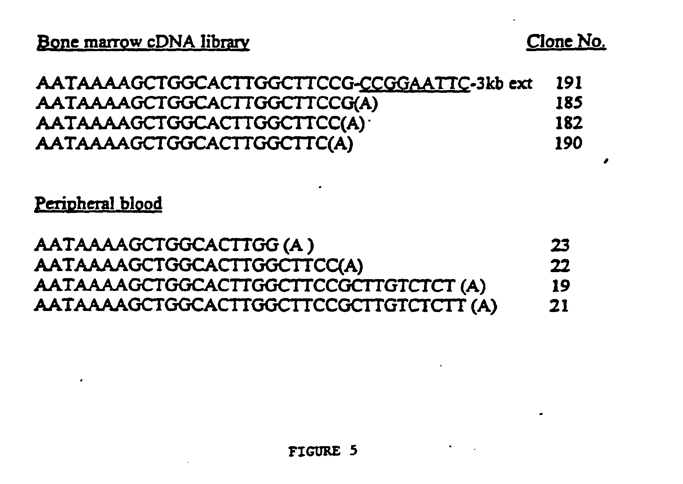

Figure 5 shows a 185 bp sequence upstream from the first possible initiation transcription site. Analysis of this region, and of exon 1, indicates that there are no TATA sequences but several other possible transcription factor binding sites were noted. At least 2 GATA-1 sites are present close to a CACCC box. Sp1 and GATA-1 sequences are present in exon 1. The 5' flanking region contains purine-rich regions. These areas of interest are shown in Figure 3.

The transcriptional activity of the 5' flanking region from -176 to -1 (Figure 3) was determined by CAT assay in transfected K562 cells as compared to the promoter-less pCAT vector. In this procedure, a PCR product spanning nucleotides -176 to -1 relative to the first cap site was subcloned into pCAT basic vector obtained from Promega Co. Cells of the erythroleukemic cell line K562 were then transfected using the lipofectin method. Construction of the CAT-vector, transfection and chloramphenicol acetyltransferase (CAT) activity were assayed following the protocol provided by Promega Co. CAT enzymatic activity was measured using [¹⁴C]-chloramphenicol (50-60 mCi/mmol, Amersham Co., Arlington Heights, IL). The reaction products were measured by liquid scintillation spectrometry and analyzing the reaction products by thin layer chromatography. As a negative control the pCAT basic vector without promoter was used. All cell extracts were normalized by protein analysis in order to compare values.

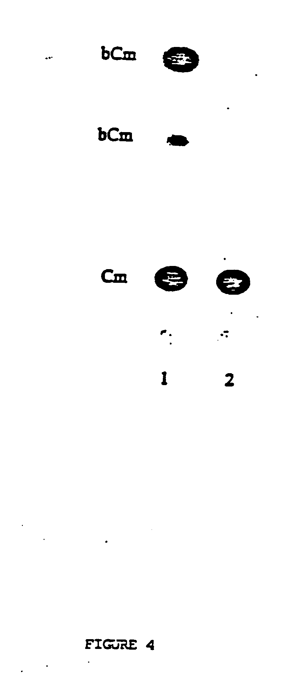

Introduced in front of a CAT reporter gene, the 5' flanking region exhibited promoter activity in the erythroleukemic cell line K562. pCAT vector with the 5' flanking region was found to express approximately 0.8 units of CAT activity per milligram of cell extract protein. A unit of CAT enzyme activity is defined as the amount of enzyme required to transfer 1 nmol of acetate of chloramphenicol in 1 minute at 37°C. Figure 4 shows CAT activity of the 5' flanking region. An autoradiogram, exposed for 48 hours, is shown. Lane 1 has pCAT vector with the 5' flanking region and lane 2 has pCAT basic vector without promoter. The radioactive butyrylated chloramphenicol is indicated as "bCm", and the unreacted chloramphenicol is indicated as "Cm".

The putative promoter region does not contain the typical TATA box usually located -25 to 30 nt relative to the cap site (Ref. 48). However, as in several erythroid specific genes, consensus sequences for GATA-1 factor were found in the promoter region. In addition, possible Sp1 and GATA-1 binding sites were noted in exon 1. It is not known whether GATA-1 and Sp1 regulate KEL gene expression but GATA-1 is common in erythroid genes (Refs. 49-55) and is known to be expressed at low levels in hematopoietic progenitor cells and up-regulated during erythroid maturation (Refs. 56-58). If these transcription factors define erythroid tissue specificity it will be in agreement with our Northern blot studies which detected Kell transcripts in bone marrow and fetal liver but not in several non-erythroid tissues (Ref. 8).

### Example 4

### Analysis of the 3' End

Exon 19 is the largest Kell exon, encoding the C-terminal 53 amino acids and containing the 3' untranslated region with a polyadenylation signal 100 bp downstream of the termination codon. Previous Northern blot analysis showed that the major Kell transcript in bone marrow and fetal liver is 2.5 kb although smaller amounts of larger mRNAs, notably 6.6 kb, also were observed (Ref. 8). In originally cloning the Kell cDNA from a human bone marrow library, we isolated a cDNA with a large (3 kb) 3' untranslated region (Ref. 25).

To determine the 3' end structure of the Kell gene, RNA was isolated from peripheral blood as described by Goosens et al. (Ref. 59) and cDNA was prepared by reverse transcription and PCR amplification using a Perkin Elmer RNA PCR kit (Roche Molecular Systems, Inc., Branchburg, NJ). First strand synthesis was initiated using an anchored oligo d(T)₁₆ primer. PCR amplification of the 3' end of Kell cDNA was performed using the anchor primer and an oligonucleotide primer from the coding sequence of Kell cDNA. The anchor antisense primer used was oligo 5'-GACTCGAGTCGACAACGTT(T)₁₆-3' (SEQ ID NO:11) and the sense primer from the 3' end coding sequence of Kell cDNA was 5'-ATGGGGAGACTGTCCTG-3' (SEQ ID NO:12).

A PCR product of about 400 bp was obtained and was subcloned and sequenced. The 3' end sequences of different sub-clones are shown in Figure 5. The base sequences, prior to the poly A region, of cDNA clones from a human bone marrow library (top) and from peripheral blood of a person with common Kell phenotype (bottom) are shown. (A) indicates the poly A region.

The 3' end sequences of four different subclones, obtained from peripheral blood are shown in the bottom portion of Figure 5. All four of the subclones had identical sequences from the termination codon to the polyadenylation signal (AATAAA). At the 3' end, the base sequences differ slightly in length before the start of the poly A sequence. The distance between the polyadenylation signal and the cleavage site is known to be variable (Ref. 60). Similar variations in 3' end sequences were observed in 3 different Kell cDNAs obtained from a human bone marrow cDNA library (Clones 185, 182 and 190).

Only one of the cDNA clones, the original full-length cDNA obtained from the bone marrow library (Clone 191), contained a large 3' untranslated region. The 3 kb untranslated fragment from this cDNA clone does not hybridize with human genomic DNA indicating that it is foreign DNA. This foreign fragment is preceded by an EcoRI site and a possible liner and may have been artificially inserted during the preparation of the library.

Sequences of the 3' segments of transcripts in peripheral blood by RNA PCR only detected short 3' untranslated regions, which varied slightly in length before the poly A tail. Similar sequences were obtained when other cDNAs from the human bone marrow library were analyzed. The larger Kell transcripts could not be amplified using the RNA PCR method because of their length, but Northern analysis indicates their presence. The occurrence of multiple transcripts with larger 3' untranslated region is not uncommon and although their function is not well understood, the 3' untranslated regions are thought to play roles in regulation of expression (Refs. 61-62).

### Examples 5-7

### DNA Preparation

In Examples 5-7, DNA was prepared from peripheral blood obtained from persons whose Kell phenotypes have been determined serologically. DNA was prepared either from 1 to 5 ml of whole blood collected with anticoagulants or, when red cells were removed by centrifugation at 1000 x g for 10 mins, from the resulting buffy coat. In both cases, the procedure described by John et al. (Ref. 63) to prepare DNA was used. Some of the DNA samples were obtained from Canadian Hutterites and serological studies of these families indicated that they were homozygous for either K1 or K2. In these samples DNA was isolated as described by Zelinski et al. (Ref. 30).

### Polymerise Chain Reaction

For Examples 5-7, the polymerase chain reaction was performed as follows: Denaturation, annealing and polymerization steps were performed in an automated thermocycler (Minicycler, MJ Research Inc., Watertown, MA). An initial cycle of 94°C for 3 min., 62°C for 1 min., and 72°C for 30 seconds. In cycles 2 through 30 the conditions were 94°C for 30 seconds, 62°C for 30 seconds, and 72°C for 30 seconds. In the last cycle the polymerization step at 72°C was extended to 10 minutes. The final concentrations of reagents were 50 mM KCl, 10 mM Tris-HCl (pH 9.0), 3 mM MgCl₂, 350 nM of each primer, 200 µM of each dNTP, 0.1% Triton-X100, 100-200 ng genomic DNA and 2.5 units of taq polymerase in a final volume of 100 µL. The "hot start" method using Ampliwax™ from Perkin Elmer (Branchburg, NJ) was employed. The amplified PCR products were separated by electrophoresis on 0.8% agarose gels and detected by ethidium bromide staining.

### Restriction Enzyme digestion

BsmI was added directly to the final PCR reaction mixture. The PCR mixture (10 µL) was optimized by BsmI digestion by adding 2 µL of 35 mM MgCl₂, 1 µL of 10 mM mercaptoethanol or 10 mM DTT, 1 µL of 10X *Bsm*I buffer, 4 µL of water, and 2 µL of 5 units/mL BsmI. The mixture were incubated for 90 min. at 65°C. The DNA in the reaction mixture was analyzed by electrophoresis in 0.8% agarose.

### Other Reagents

Taq DNA polymerase and dNTPs were purchased from Promega Co. (Madison, WI). X-Gal was purchased from Appligene Inc. (Pleasanton, CA). The DNA 1 kb ladder standards, DH5αF' strain E. *coli* competent cells, and the low melting agarose were purchased from Bethesda Research Laboratories (Gaithersburg, MD). T4 DNA ligase and BsmI were purchased from New England Biolabs (Beverly, MA). pT7 blue (R) plasmid vector was purchased from Novagen (Madison, WI). Quick Spin™ Column (G-50 Sephadex) for DNA purification was purchased from Boehringer Mannheim, Inc. (Indianapolis, IN).

### Example 5

### Comparison of K1 and K2 DNA Sequences

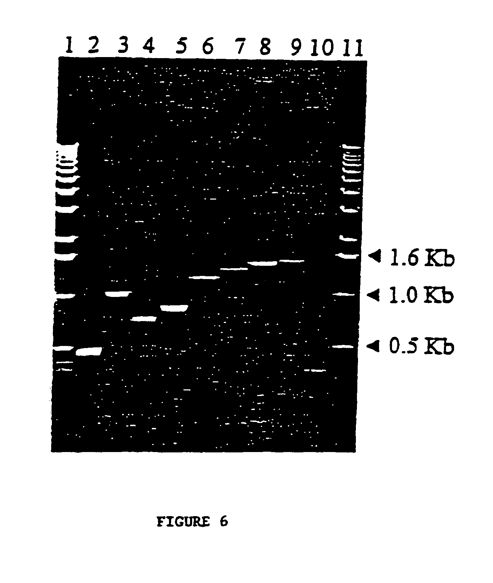

Nine pairs of forward and reverse primers were used to amplify the 19 KEL exons. These primer pairs were selected to cover the open reading frames of the exons. The sequences of the primers and the target exons (spanning regions) are shown in Table 1. Genomic DNA from a homozygous K1 person was used as template DNA. The PCR products were separated by electrophoresis on 0.8% agarose gels and stained with ethidium bromide. In all cases single products ranging in size from 0.48 to 1.5 kb were obtained. Figure 6 illustrates the PCR amplification of the KEL exons. Lanes 1 and 11 in Figure 6 are 1 kb DNA ladder standards. Lanes 2 to 10 contain PCR products of the primer pairs PCR 1, 2, 3, 4, 5, 6, 7, 8 and 9, in that order.

**Table 1, Primers Used in PCR of KEL Exons**

| PCR | Primers | SEQ ID NOS: | Target Exons | Product (kb) |
|---|---|---|---|---|
| 1 | 5′-CAG TCC TCC GAA TCA GCT CCT AGA-3′ 5′-CTC TTG GCT CCA GAG AGT TCC CAT-3′ | 13 | 1*2* | 0.48 |
| | | 14 | | |
| | | | | |
| 2 | 5′-GAA GGT GGG GAC CAA AGT GAG GAA-3′ 5′-ACA GGG TTT GGA GCA GTC ATG GTC-3′ | 15 | 2*,3,4 | 1.0 |
| | | 16 | | |
| | | | | |
| 3 | 5'-TTT AGT CCT CAC TCC CAT GCT TCC-3' 5'-TAT CAC ACA GGT GTC CTC TCT TCC-3' | 4 | 5,6 | 0.74 |
| | | 5 | | |
| | | | | |
| 4 | | 17 | 7,8,9 | 0.8 |
| | 5'-ATA TTC CCC ACC TCC CCA CAC CTG-3' 5'-ATC TAC GGT GCT CAG GCT CTC CTC-3' | 18 | | |
| | | | | |
| 5 | | 19 | 10 | 1.2 |
| | 5′-GGA AGC ATG GGA GTG AGG ACT AAA-3′ 5′-TGG CAT CCA TGG TAC CTC ATG GAA-3′ | 20 | | |
| | | | | |
| 6 | | 21 | 11 | 1.3 |
| | 5′-GAG GCT TTT GAA ACC CCA GGA TGA-3′ 5′-TTC CCC AGC CAC CTG CCA TCT CAT-3′ | 22 | | |
| | | | | |
| 7 | | 23 | 12,13,14,15 | 1.4 |
| | 5′-CCC TTT TCC AAG GGT CAG AAG CTG-3′ 5′-GGG CTT ATT TGA CCC CCA GAA TCT-3′ | 24 | | |
| | | | | |
| 8 | | 25 | 14*,15,16,17,18 | 1.5 |
| | 5′-CCT AAT CCC TGG ATG CCT GCC TGT-3′ 5′-CAG TGA GGA CAT CTG CAG AAG AGG-3′ | 26 | | |
| | | | | |
| 9 | 5'-TCC TGT GGA CCC TCC CCC TTC AAT-3' 5'-GGG CGG AAG CCA AGT GCC AGC TTT T-3' | 27 | 19* | 0.33 |
| | | 28 | | |

The first primer is the forward sequence and the second is the antisense primer. The 2G bases at the 5' end of antisense primer of PCR are not in the gene and were added to create an SmaI site for another purpose. Some PCR products did not cover the entire exons and those exons are marked with asterisks. The PCR products of exons 1 (PCR1) and 19 (PCR 9) contain the entire translated regions. Exon 2 was spanned by overlapping PCR products (PCR 1 & 2) and exon 14 was covered by PCR 7.

### Example 6

The PCR products obtained in Example 1 were sequenced using the following protocol: The PCR products, separated by electrophoresis on low 2.8% melting agarose, were eluted, ligated to pT7 Blue(R) plasmid vector and transformed in DH5αF' strain of *E*. coli. Plasmid DNA was prepared on a small scale by the alkali lysis method and purified with a Quick Spin™ (Sephadex G50) column. Standard molecular biology procedures were employed, such as are described in detail in Sambrook et al. (Ref. 42). DNA sequencing was performed by an automated system (Applied Biosystems, Model 373A, Version 1.2.0).

The sequenced products were compared to the previously described sequence of K2 cDNA (Ref. 25). It was unexpectedly found that the sequence of K1 DNA encodes an amino acid sequence identical to that of K2 DNA, except for a single base change. This difference between K1 and K2 occurs as a shift from C to T at nucleotide 701 in exon 6.

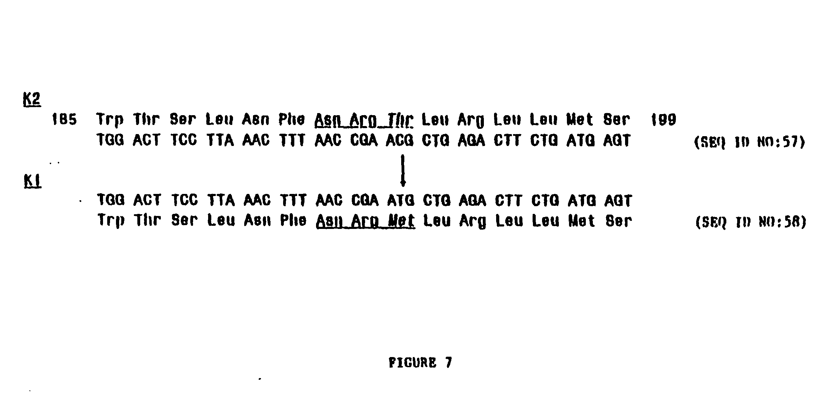

The PCR product PCR3 (Table 1), which spans exons 5 and 6, had a single base difference when compared to K2 DNA. The base sequences of a portion of exon 6, and the amino acids which they encode, are shown in Figure 7. The sequence of K2 DNA is on the top and K1 DNA on the bottom. In the PCR3 product, which was 740 bp in length, there was a single cytosine (C) to thymine (T) substitution, corresponding to position 701 of the Kell cDNA. The C to T substitution is marked in bold letters and is highlighted with an arrow. This change predicts a threonine to methionine change at a consensus N-glycosylation site (Asn.X.Thr). An N-linked glycosylation motif in K2, and the disrupted motif in K1, are underlined. Since this single base change was the only difference between K1 and K2 encoding a change in an amino acid, this observation suggested that the threonine to methionine change (Thr→ Met) at position 193 would prevent N-glycosylation at asparagine 191 in proteins expressing K1, thus identifying the K1/K2 polymorphism.

### Example 7

### Confirmation of K1/K2 Polymorphism by BsmI Analysis

The C to T substitution at nucleotide position 701 (nt 701) in exon 6 creates a new sequence including the sequence 5'-GAATGCT-3', which is known to define a restriction enzyme site specific for BsmI. As a result, treatment of the 740 bp PCR product which spans exons 5 and 6 (PCR3) with BsmI was hypothesized to provide a means by which to differentiate K1/K2 genotypes. In a PCR method employing digestion with EsmI, the K1/K1 genotype should yield 2 fragments, of 540 and 200 bp; the K2/K2 genotype should yield the uncut 740 bp PCR product and K1/K2 heterozygotes should yield 3 fragments of 740, 540 and 200 bp. To confirm that this base change does in fact identify the K1 genotype, this region was analyzed in 42 different persons of known K1/K2 phenotype.

The PCR3 primer pair (Table 1) was used to generate PCR products (740 bp) by PCR amplification of DNA obtained from various Kell phenotypes. After amplification, the samples were treated with BsmI and separated by electrophoresis on 0.8% agarose gels.

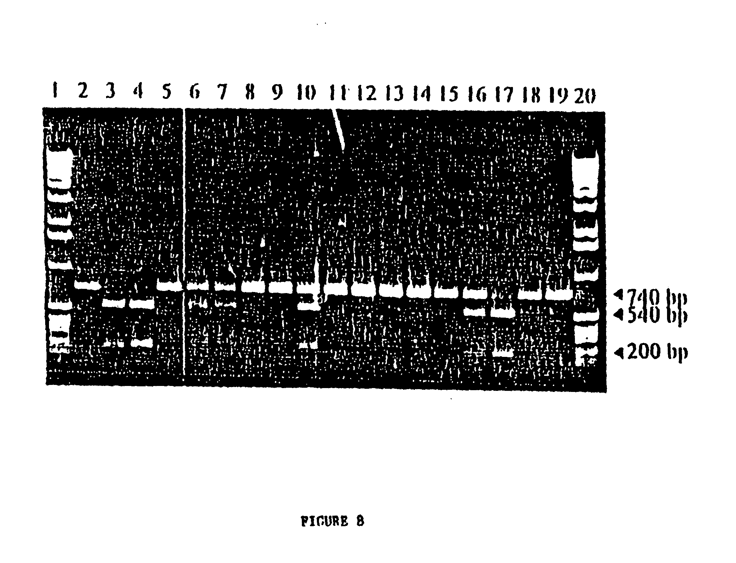

**Figure 8 illustrates the results obtained in the above-described experiment. The gel lanes are defined as follows:**

| Lane | Phenotype | Lane | Phenotype |
|---|---|---|---|
| 1 | DNA Standard | 11 | K:-1,2,3,-4 |
| 2 | Untreated PCR3 | 12 | K:-1,2,6,-7 |
| 3 | R:1,-2 | 13 | McLeod |
| 4 | K:1,-2 | 14 | K:-1,2,10 ' |
| 5 | K:-1,2 | 15 | K:-1,2,14,24 |
| 6 | K:1,2 | 16 | K:1,2 |
| 7 | K:1,2 | 17 | K:1,-2 |
| 8 | K:-1,2 | 18 | K:-1,2 |
| 9 | Kₒ | 19 | K:-1,2 |
| 10 | K:1,2 | 20 | DNA Standard |

Lanes 1 and 20 contain 1 kb ladder DNA standards. Lane 2 is untreated PCR3 from K1 homozygotes. Lanes 3 to 19 are treated with BsmI. All K2 homozygote (K:-1,2) samples gave uncut 740 bp products. K1 homozygotes (K:1,-2) yielded only 540 and 200 bp fragments. K1/K2 heterozygotes (K:1,2) had 3 bands, the uncut 740 and the smaller 540 and 200 bp products.

Of the 42 DNA samples tested, 12 were either K1 or K2 homozygotes, 6 were K1/K2 heterozygotes and 24 were K:-1,2 phenotypes, but contained low prevalence or rare Kell phenotypes. These included K3, K6, K10, Kₒ, K14/K24 heterozygote, and a McLeod phenotype. In 40 of the 42 cases the BsmI genotyping agreed with the Kell phenotypes which were determined by serological analysis of red cells. In two cases, genotyping identified one of the samples as K:-1,2 homozygote and the other as a K:1,2 heterozygote. These two samples were, however, serologically identified as having "weak" K1 phenotypes.

None of the other low prevalence or rare Kell phenotypes listed above had the C to T base substitution in exon 6, indicating that this change is specific for the K1/K2 polymorphism.

### Example 8

In a method of differentially determining K1/K2 genotype, the polymerase chain reaction was employed to test samples of genomic DNA using a unique primer mixture. The primer mixture included the following primers:

| | | |
|---|---|---|
| MK1R | ATA CTG ACT CAT CAG AAG TTT CAG CA | (SEQ ID NO:1) |
| MK2F | TGG ACT TCC TTA AAC TTT AAC TGA AC | (SEQ ID NO:3) |
| EI5F | TTT AGT CCT CAC TCC CAT GCT TCC | (SEQ ID NO:4) |
| EI6R | TAT CAC ACA GGT GTC CTC TCT TCC | (SEQ ID NO:5) |

The MK1R primer is specific for K1 DNA, producing a PCR product 540 bp in length. The MK2F primer is specific for K2 DNA, and produces a PCR product 240 bp in length. The other primers EI5F and EI6R are K1/K2 specific, producing PCR products 740 bp in length, and are used as an internal control.

The primer mix included the primers in the following concentrations: 20 µg/µL MK1R; 20 ng/µL MK2F; 30 ng/µL EI5F; and 30 ng/µL EI6R. EI5F and EI6R primers are used in the PCR for two reactions, while the MK1R and MK2F primers are each used for only one reaction. The concentrations of the primers were adjusted to ensure sufficient quantities of the EI5F and EI6R primers for the duration of the reaction.

Blood samples were obtained from human volunteers. DNA was isolated from leukocytes present in the buffy coat of blood samples using the method described elsewhere herein.

Reaction tubes were set up including one Ampliwax™ and 25 µL of a first reagent cocktail including: 2.5 µL 10X buffer (Promega Co., Madison, WI); 4 µL dNTP; 3 µL 25 mM MgCl₂; 7 µL primer mix; and 8.5 µL H₂O, to give a preliminary reaction mixture volume of 25 µL. These preliminary mixtures were incubated a 80°C for 5 min. and cooled to room temperature for 5 min. before proceeding. To each of the tubes were added 23 µL of a second reagent cocktail including: 2.5 µL 10X buffer; 3 µL 25 mM MgCl₂; 17 µL H₂O; and 0.5 µL *taq* DNA polymerase (Promega Co., 5 units/µL) and 100 ng of isolated genomic DNA in 2 µL H₂O.

The PCR reaction was performed using the following cycling program. The initial cycle comprised 94°C for 3 minutes, 62°C for 1 minute, and 72°C for 30 seconds. Cycles 2-30 each included 94°C for 30 seconds, 62°C for 30 seconds, and 72°C for 30 seconds. In the last cycle, the polymerization step was 72°C was extended to 10 minutes.

Following completion of the PCR, the amplified products were resolved using ethidium bromide/agarose gel electrophoresis as described in the art.

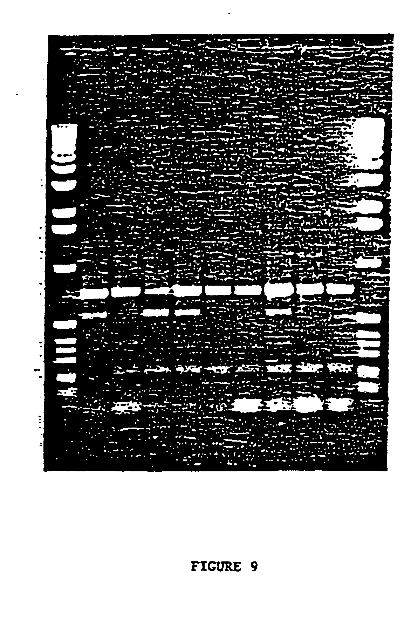

The results are of this procedure are shown in Figure 9. Lanes 1 and 11 are identical control samples including mixed DNA fragments of known size. Lanes 2-4 represent internal control samples from subjects in whom the K1/K2 genotype was already known. Control Lane 2 shows the presence of 540 bp and 740 bp fragments, indicating that the subject is homozygous K1 (K:1,-2). Control Lane 3 shows the presence of 200 bp and 740,bp fragments, indicating that the subject is homozygous K2 (K:-1,2). Control Lane 4 shows the presence of each of the K1/K2 fragments, i.e., 200 bp, 540 bp, and 740 bp, indicating that the subject is heterozygous (K:1,2).

Lanes 5-7 in Figure 9 represent samples from a family of test subjects: Lanes 5 and 6 represent parents and Lane 7 represents the fetus. A comparison of the test bands with the control bands reveals that the parent represented in Lane 5 is heterozygous (K:1,2) and that the parent represented in Lane 6 is homozygous K2 (K:-1,2). The fetus is clearly homozygous K2 (K:-1,2).

Lanes 8-10 in Figure 9 represent samples from another family of test subjects. The first parent, represented in Lane 8, is heterozygous (K:1,2), while the second parent, represented in Lane 9, is homozygous K2 (K:-1,2). The fetus, represented in Lane 10, is identifiable as being homozygous K2 (K:-1,2).

The experimental data provided in Examples 7 and 8 clearly demonstrate the diagnostic efficacy of the method of the invention. The examples show that individuals of each of the three K1/K2 genotypes can be distinguished positively by virtue of the differences between K1 DNA and K2 DNA. More particularly, the newly identified locus of K1/K2 polymorphism can be exploited by means of amplification of Kell DNA using K1- and K2-specific nucleic acid probes, as well as by differential digestion of K1/K2 DNA with a restriction enzyme.

### Example 9

The molecular basis for the K6/K7 polymorphism has now unexpectedly been found, showing that it is due to a point mutation in exon 17 leading to proline (K6) being encoded instead of leucine (K7) at amino acid position 597 of the Kell protein. This amino acid substitution is in close proximity to the zinc-binding putative enzyme active site of Kell protein.

### DNA Preparation.

Leukocyte DNA was prepared from peripheral blood of eight unrelated individuals with K6 phenotype, as described in Examples 5-7, based on a procedure described by John et al. (Ref. 63).

### Polymerase chain Reaction (PCR).

The forward and reverse primers used to copy and amplify the 19 exons of the *KEL* gene were, for the most part, the same as those previously described elsewhere herein. In a few instances, other primers were designed to produce smaller PCR products to facilitate sequencing. The forward and reverse primers used to amplify an 807 bp segment from exons 17 and 18 are 5'-CTCACCTAGGCACCACCAACCCTA-3' (SEQ ID NO:64) and 5'-CAGTGAGGACATCTGCAAGAGG-3' (SEQ ID NO:26), respectively. The PCR conditions used were the same as previously described in Examples 5-7 above, except that the number of cycles was increased to 35.

### DNA Sequencing.

The PCR, products were separated by gel electrophoresis on 0.8% low melting agarose gels. After elution the DNA was either directly sequenced or subcloned into pT7Blue(R)T plasmid vector (Novagen, Madison, Wisconsin), and transformed in DH5αF' strain of *E. coli* (Gibco BRL Life Technologies, Gaithersburg, Maryland). If subcloned, the plasmid DNA was prepared in a small scale by the alkali lysis method and purified with a Quick spin™ (Sephadex™ G50) column. Standard molecular biology procedures were employed. DNA sequencing was performed by an automated system (Model 373A, Version 1-2.0, Applied Biosystems, Foster City, CA).

### Restriction Enzyme Digestion of PCR Product.

A short 111 bp sequence of exon 17, predicted to contain the point mutation for the K6 genotype, was copied and amplified by PCR. only a short segment of exon 17 was copied because exon 17 contains other neighboring *Mnl*I cleavage sites. The following oligonucleotide primers were used for the PCR reaction, 5'-CTCACCTAGGCAGCACCAACCCTA-3' (SEQ ID NO: 64) (forward primer, same as described in Example _), and 5'-TTACCTGGAGGGCATGGTTGTCACT-3' (SEQ ID NO:65) (reverse primer) . The PCR product (10 µL of final reaction mixture) was treated with 10 Units of *Mnl*I (New England Biolabs, Beverly, Massachusetts) and incubated at 37°C for 90 minutes. The final reaction volumes was 20 µL. The DNA was then separated by electrophoresis on 0.7% agarose gels, supplemented with 1.65% GelTwin™ brand surfactant[?] (J.T. Baker, Philipsburg, New Jersey).

### Comparison of K6 and K7 DNA sequences.

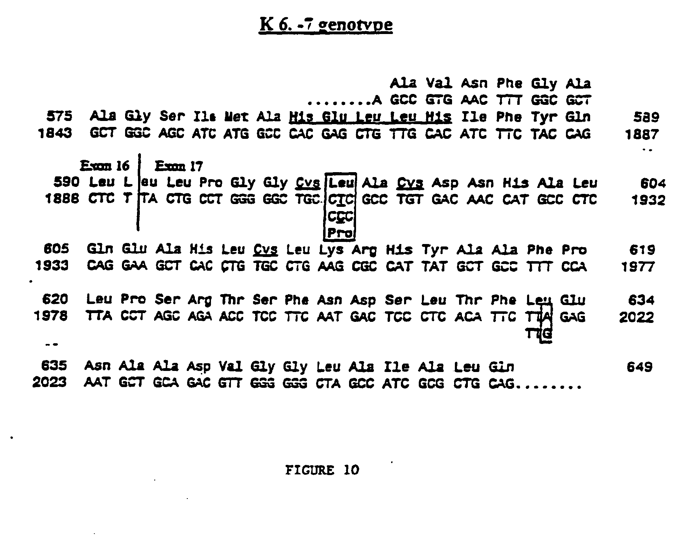

The base sequences of the PCR products corresponding to the 19 exons of K:6,-7 DNA were compared to a "wild-type" DNA (K:-6,7), which expresses the high incidence Kell phenotypes including K7 (Ref. 43). Two discrete base substitutions were noted, both occurring in exon 17. The changes are shown graphically in Figure 10. At nt 1910, a T to C change was observed which results in a proline being encoded instead of leucine at amino acid position 597. Another base substitution, A to G, was observed at nt 2019, but this change does not cause the encoding of a different amino acid.

### Confirmation of K6/K7 Polymorphism by MnlI Digestion.

The T to C substitution at nt 1910 eliminates a recognition site for the restriction endonuclease *Mnl*I. Thus, wild-type K7 DNA is cleaved at this point when exposed to *Mnl*I, while the low frequency K6 DNA is not affected. This distinct feature may be used to analyze for this point mutation and to determine whether this substitution identifies the K6 genotype. A 111 bp sequence, which spans this region of exon 17, was obtained by PCR of genomic DNA from eight unrelated K:6,-7 and K:-6,7 persons, and treated with *Mnl*I. The K7 (wild-type) DNA contains the *Mnl*I site and upon treatment with *Mnl*I yields a 91 bp and 20 bp products. On the other hand, the K6 PCR product is not cleaved by *Mnl*I, and the 111 bp product remains uncut. Thus, a heterozygote yields both the uncut PCR product and the two smaller bands.

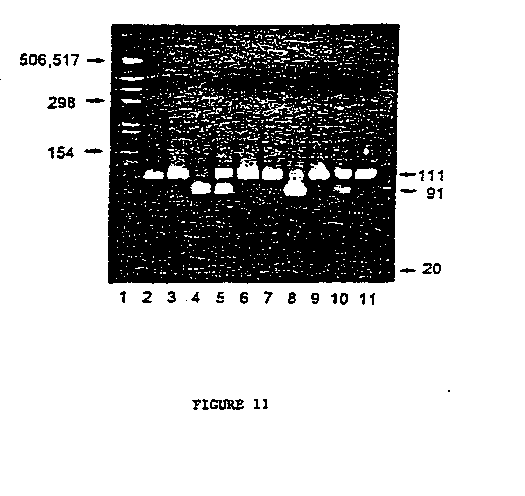

The results are shown in Figure 11. PCR products from seven unrelated K6 phenotype persons all showed 111 bp bands. Four examples are shown (Lanes 3, 6, 7, 9). PCR products from all K7 phenotype persons showed a smaller 91 bp product (Lanes 4 and 8), but the 20 bp product, because of its small size, was difficult to detect by ethidium bromide staining. The K:6,7 heterozygotes (Lanes 5 and 10) showed both the uncut 111 bp band and the smaller 91 bp band.

Chimpanzee red cells carry the K6 (Js^{a}) antigen (Ref. 27). A single DNA sample from a chimpanzee was analyzed and it also yielded the 111 bp uncut PCR product, a pattern which is consistent with a K:6,-7 genotype. This result is shown as Lane 11 in the gel shown in Figure 11.

### Example 10

The K6/K7 polymorphism was explored further and a second restriction enzyme cleavage site was identified in association with the point mutation at nt 2019. While this is a silent mutation, it may be exploited according to the invention since it is consistently associated with the expressed mutation at nt 1910.

DNA was prepared from peripheral blood of the seven individuals identified in Example 9, as described therein. The PCR and DNA sequencing were performed as described in Example 9. The 807 bp PCR product was digested with DdeI. Ten microliters (10 µL) of the PCR product was incubated with 10-20 units of *Dde*I (New England Biolabs, Beverly, Massachusetts) at 37°C for 90 minutes, and the enzyme-digested mixture was separated on a 0.7% agarose gels supplemented with 1.65% GelTwin™, as described in Example 9.

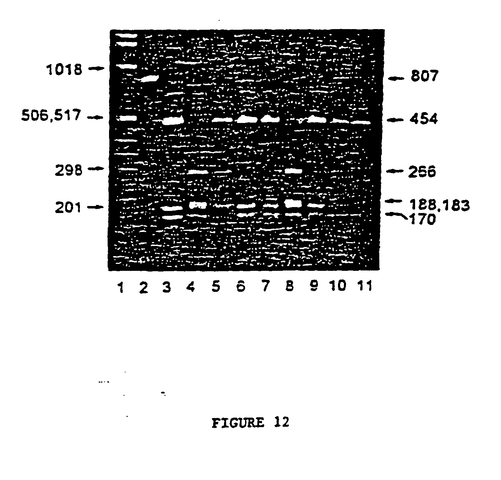

The PCR product was found to have several *Dde*I sites, and multiple bands were obtained upon digestion. DNA from four K6 phenotype individuals yielded three prominent bands of 454, 183, and 170 bp (Figure 12, Lanes 3, 6, 7, and 9). By contrast, the K& phenotypes yielded four bands (266,188,183, and 170 bp), because the 454 bp band was further digested to 266 and 188 bp products (Lanes 4 and 8). the 188 and 183 bp products are not well separated, and sometimes migrate together. Two heterozygotes (Lanes 5 and 10) could be distinguished from the K6 and K7 homozygotes, because they yielded five bands (454, 266, 188, 183, and 170 bp).

One sample of chimpanzee DNA was also digested using DdeI. As noted above in Example 9, chimpanzee red cells carry type K:6,-7. As shown in Figure 12, the 454 bp product was uncut by DdeI (Lane 11), again consistent with a K:6,-7 genotype.

A comparison of the base sequence of the coding region of the KEL gene of K6 and K7 DNA showed only 2 base differences, both occurring in exon 17. See Figure 10. only one of these base substitutions encoded a different amino acid. A T to C change at nt 1910 changed a leucine to a proline at amino acid residue 597. The other base substitution at nt 2019 is a silent mutation which retains leucine at that position. Both of the base substitutions at nt 1910 and nt 2019 are common to K:6,-7 genotypes, since analysis of the seven unrelated individuals described in Examples 9 and 10 showed these base changes. As expected, K:6,7 heterozygotes possessed both the wild-type and the K:6,-7 sequences.

The pentameric consensus sequence, HEXXH, which Kell protein shares with a family of zinc endopeptidases occurs at amino acid residues 581 to 585. Thus, this putative active site is in close proximity to residue 597 which has the leucine (K7) to proline (K6) change. We cannot predict whether this amino acid change influences the putative enzymatic activity of Kell, but its proximity cannot be ignored. It is also of interest that the leucine to proline change occurs at a residue which is located between two cysteine residues (see Figure 10) yielding a Cys.Pro.Ala.Cys sequence in K6, and a Cys.Leu.Ala.Cys sequence in K7. Because of their distinctive molecular structure, proline residues can induce local structural changes in proteins, and can change the geometry of helices. In this case, because it is situated between two cysteine residues, proline could cause rearrangements of disulfide bonds, thus markedly changing the secondary and tertiary structure of the protein and exposing new epitopes. A computer assisted Chou-Fasman analysis of the secondary structure of Kell protein indicates that substituting proline for leucine at residue 597 would, in fact, alter the turns predicted for that region.

It should be noted that Kell has 16 cysteine residues, with 15 of these residues occurring in the extracellular domain, and one occurring in the membrane-spanning region. This indicates extensive folding of the protein, which is in agreement with early biochemical studies showing inactivation of Kell antigen by reducing reagents. Of all the Kell antigens, K6 and K7 are the ones which are inactivated with the lowest concentrations (1-2 mM) of dithiothreitol (DTT). Other Kell antigens such as K1, K2, K3 and K21 require much higher concentrations (100-200 mM) of DTT for inactivation.

An immunological procedure, termed MAIEA, has been used to map the spatial relationships between Kell epitopes. This method has predicted that K6/K7 is close to the K1/K2 epitope in the native Kell protein. The point mutation which distinguishes the K1/K2 polymorphism occurs at amino acid residue 193, while that for K6/K7 is linearly distant, occurring at amino acid 597. This apparent discrepancy again points to the folded nature of Kell protein and that the epitopes can be far removed from the points of mutation.

The experimental data provided in Examples 9 and 10 again show the diagnostic efficacy of the method of the invention. Specifically, individuals of having various K6/K7 genotypes can be distinguished positively on the basis of differences between K6 DNA and K7 DNA. More particularly, the newly identified locus of K6/K7 polymorphism can be exploited by means of amplification of Kell DNA using K6- and K7-specific nucleic acid probes, as well as by differential digestion of K6/K7 DNA with a restriction enzyme.

### Example 11

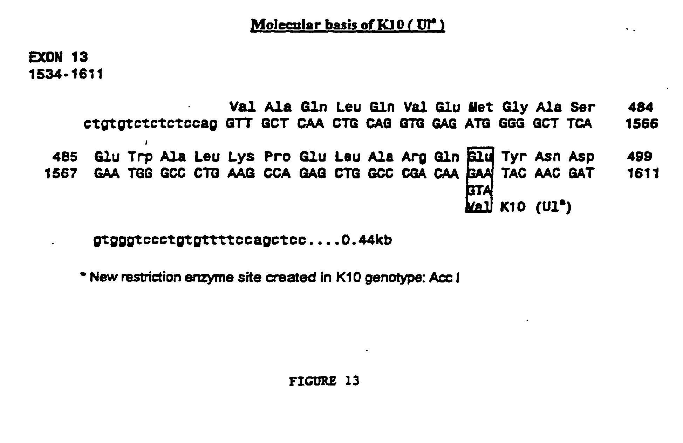

Further studies revealed that a single nucleotide substitution at nt 1601 in exon 13 of the KEL gene is associated with K10 genotype. The molecular basis of K10 is shown in Figure 13, which provides the nucleotide and amino acid sequences of wild-type Kell DNA in the K10 polymorphism locus, as well as the point mutation (A 1601 T) which causes a change in amino acid sequence (Glu 495 Val). The point mutation at nt 1601 results in the K10 phenotype.

RFLP analysis was performed in general accordance with the methods described elsewhere herein. It was found that the nucleotide substitution creates a new cleavage site for the restriction enzyme *Acc*I, permitting differential detection of amplification products from K10 and wild-type DNA and consequent differential determination of K10 genotype.

### Example 12

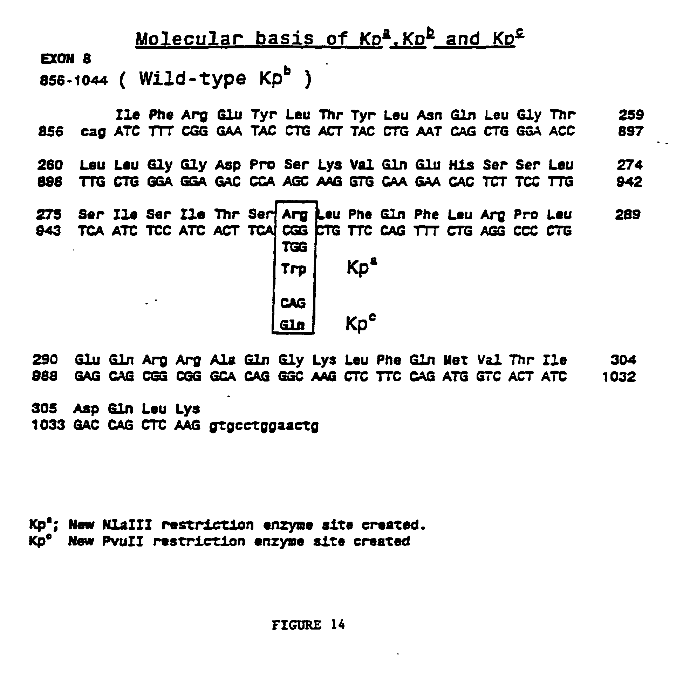

Analysis of the K3/K4/K21 polymorphism locus was performed to establish whether RFLP methods could be used to determine K3/K4/K21 genotype. Studies revealed unexpectedly that two discrete nucleotide substitutions in a single codon are associated with the polymorphism of the K3/K4/K21 locus. The molecular basis of K4 is shown in Figure 14, which provides the nucleotide and amino acid sequences of K4 DNA, as well as the point mutations which causes changes in amino acid sequence. The point mutation at nt 961?(C → T) cause a change in amino acid sequence (Arg 281 Trp), resulting in the K3 phenotype. The point mutation at nt 962 (G → A) cause a change in amino acid sequence (Arg 281 Gln), resulting in the K21 phenotype.

RFLP analysis was performed in general accordance with the methods described elsewhere herein. It was found that the C 961 T nucleotide substitution creates a new cleavage site for the restriction enzyme *Nl*aIII, permitting differential detection of amplification products from K3 and wild-type K4 DNA and consequent differential determination of K3/K4 genotype. It was also found that the G 962 A nucleotide substitution creates a new cleavage site for the restriction enzyme PvuII, permitting differential detection of amplification products from K21 and wild-type K4 DNA and consequent differential determination of K21/K4 genotype. Together, the discovery of these restriction sites now enables a method for directly determining K3/K4/K21 genotype.

### REFERENCES

1. Giblett ER, "A critique of the theoretical hazard of inner- vs. intra-racial transfusion" Transfusion 1:233 (1961).
2. Mayne KM, Bowell PJ, and Pratt GA, "The significance of anti-Kell sensitization in pregnancy" Clin Lab Haematol 12:379-385 (1990).
3. Duguid JKM, and Bromilow IM, "Haemolytic disease of the newborn due to anti-K" Vox Sang 58:69 (1990).
4. Moncharmont P, Juron-Duprax F, Doillon M, Vignal M, and Debeaux V, "A case of hemolytic disease of the newborn infant due to anti-K (Cellano)" Acta Haematol 85:45 (1991).
5. Constantine G, Fitzgibbon N, and Weave JB, "Anti-Kell in pregnancy" Brit J Obs Gyn 98: 943 (1991).
6. Leggat HM, Gibson JM, Barron SL, and Reid MM, "Anti-Kell in pregnancy" Brit J Obs Gyn 98:162 (1991).
7. Bowman JM, Pollock JM, Manning FA, Harman CK, and Menticoglou S, "Maternal Kell blood group alloimmunization" Obstetrics and Gynecology 79:239 (1992).
8. Lee S, Zambas E, Marsh WL, and Redman CM, "The human Kell blood group gene maps to chromosome 7q33 and its expression is restricted to erythroid cells" Blood 81:2804 (1993).
9. Zelinski T, Coghlan G, Myal Y, Shiu RPC, Phillips S, White L, and Lewis M, "Genetic linkage between the Kell blood group system and prolactin-inducible protein loci: Provisional assignment of KEL to chromosome 7" Ann Hum Genet 55:137 (1991).
10. Purohit KR, Weber JL, Ward LJ, and Keats BJB, "The Kell blood group locus is closed to the cystic fibrosis locus on chromosome 7" Hum Genet 89:457 (1992).
11. Murphy MT, Morrison N, Miles JS, Fraser RH, Spurr NK, and Boyd E, "Regional chromosomal assignment of the Kell blood group locus (KEL) to chromosome 7q35-q35 by fluorescence in situ hybridization: Evidence for the polypeptide nature of antigenic variations" Hum Genet 91:585 (1993).
12. Giblett ER, and Chase J, "Js" a 'new' red cell antigen found in negroes: evidence for an eleventh blood group system" Brit J Haematol 5:319-26 (1959).
13. Furuhjelm U, Nevanlinna HR, Nurkka R, Gavin J, Tippett P, Gooch A, and Sanger R, "The blood group antigen U1a (Karhula)" Vox Sang 15:118-24 (1968).
14. Marsh WL, "Blood groups of human red cells in clinical practice of blood transfusion" (Petz, LD and Swisher SN, eds) pp. 79-130, Churchill-Livingstone, New York (1981).
15. Marsh WL and Redman CM, "Recent developments in the Kell blood group system" Trans Med Rey 1:4 (1987).
16. Marsh WL and Redman CM, "The Kell blood group system: a review" Transfusion 30:151 (1990).
17. Redman CM and Marsh WL, "The Kell antigens and McLeod red cells" in Protein Blood Group Antigens of the Human Red Cell:Structure. Function and Clinical Significance. (Agre PC and Cartron JP, eds.) pp 53-69, Johns Hopkins University Press, Baltimore, MD (1992).
18. Redman CM and Marsh WL, "The Kell blood group system and the McLeod phenotypes" Seminars in Hematology 40:309 (1993).
19. Redman CM, Marsh WL, Mueller KA, Avellino GP, and Johnson CL, "Isolation of Kell-active protein from the red cell membrane" Transfusion 24:176 (1984).
20. Wallas C, simon R, Sharpe MA, and Byler C, "Isolation of Kell-reactive protein from red cell membranes" Transfusion 26:173 (1986).
21. Redman CM, Avellino G, Pfeffer SR, Mukherjee TK, Nichols M, Rubinstein P, and Marsh WL, "Kell blood group antigens are part of a 93,000 Dalton red cell membrane protein" J Biol Chem 261:9521 (1986).
22. Jaber A, Blanchard D, Goossens D, Bloy C, Lambin P, Rouger P, Salmon C, and Cartron JP, "Characterization of blood group Kell (K1) antigen with a human monoclonal antibody" Blood 73:1597 (1989).
23. Jaber A, Loirot MJ, Willem C, Bloy C, Cartron JP, and Blanchard D, "Characterization of murine monoclonal antibodies directed against the Kell blood group glycoprotein" Brit J Haematol 79:311 (1991).
24. Parson SF, Gardner B, and Anstee DJ, "Monoclonal antibodies against Kell glycoprotein: Serology, immunochemistry and quantification of antigen sites" Transfusion Medicine 3:137 (1993).
25. Lee S, Zambas E, Marsh WL, and Redman CM, "Molecular cloning and primary structure of Kell blood group protein" Proc Natl Acad Sci (USA) 88:6353 (1991).
26. Petty AC, Daniels GL, and Tippett P, "Application of the MAIEA assay to the Kell blood group system" Vox Sang 66:216 (1994).
27. Redman CM, Lee S, Ten Bokkel Huinink D, Rabin, BI, Johnson CL, oyen R, and Marsh WL, "Comparison of human and chimpanzee Kell blood group system" Transfusion 239:486-490 (1989).
28. Branch D, Muensch H, Sy Siok Hian A, and Petz D, "Disulfide bonds are a requirement of Kell and Cartwright (Yta) blood group antigen integrity" Br J Haematol 54:573-578 (1993).
29. Jongeneel CX, Bouvier J, and Bairoch A, "A unique signature identifies a family of zinc-dependent metallopeptidases" FEBS Letters 242:211 (1989).
30. Zelinski T, "The use of DNA restriction fragment length polymorphisms in conjunction with blood group serology" Transfusion 31:762-770 (1991).
31. Bause E, "Structural requirements of N-glycosylation proteins" Biochem J 209:331-336 (1983).
32. Telen MJ, Le Van Kim C, Guizzo ML, Cartron JP, and Colin Y, "Erythrocyte Webb-type glycophorin C variant lacks N-glycosylation due to an asparagine to serine substitution" Am J Hematol 37:51-52 (1991).
33. Chang S, Reid M, Conboy J, Kan Y, and Mohandas N, "Molecular characterization of erythrocyte glycophorin C variants" Blood 77:644-648 (1991).
34. Innis MA, Gelfand DH, Sninsky JJ, and White TJ, PCR Protocols: A Guide to Methods and applications, Academic Press, Inc., San Diego (1990).
35. Landegren U, et al., Science 241:1077 (1988).
36. Barany F, PCR Methods and Applications 1:5 (1991).
37. Narang et al., Beth Enzymol 68:90 (1979.
38. Brown et al., Meth Enzymol 68:109 (1979).
39. Beaucage et al., Tetrahedron Lett 22:1859 (1981).
40. Glick BR and Pasternak JJ, Molecular Biotechnology: Principles and Applications of Recombinant DNA, American Society for Microbiology Press, Washington, D.C. (1994).
41. Walker RH et al., eds., Technical Manual, 10th ed., American Association of Blood Banks, Arlington, Virginia (1990).
42. Sambrook J, Fritsch EF, and Maniatis T, Molecular Cloning: A Laboratory Manual, 2d. ed., Cold Spring Harbor Laboratory, NY (1989).
43. Lee S, Zambas E, Green ED, and Redman C, "Organization of the gene encoding the human Kell blood group protein" Blood 85:912-16 (1995).
44. Gerard NP, Bao L, Yiao-Ping H, Eddy RL, Shows TB, and Gerard C, "Characterization of the human C5a receptor gene" Biochemistry 32:1243 (1993).
45. Letarte M, Vera R, Tran JBL, Addis JBL, Ouizuka RJ, Quackenbush EJ, Jongeneel CY, and McInnes RR, "Common acute lymphocytic leukemia antigen is identical to neutral endopeptidase" J Exp Med 168:1247 (1988).
46. Shipp MA, Vijayaraghavan J, Schmidtt EV, Masteller EL, D'Adamio L, Heish LB, and Reinherz EL, "Common acute lymphoblastic antigen (CALLA) is active neutral endopeptidase 24.11 (enkephalinase): Direct evidence by cDNA transfection analysis" Proc Natl Acad Sci USA 86:297 (1989).
47. D'Adamio L, Shipp MA, Masteller EL, and Reinherz EL, "Organization of the gene encoding common acute lymphoblastic leukemia antigen (neutral endopeptidases 24.11):Multiple mini exons and separate 5' untranslated region" Proc Nail Acad Sci USA 86:7103 (1989).
48. Bucher P, and Trifonov EN, "Compilation and analysis of eukaryotic POL II promoter sequences" Nucleic Acids Res. 14:10009 (1986).
49. Evans T, Reitman M, and Felsenfeld G, "An erythrocyte-specific DNA-binding factor recognizes a regulatory sequence common to all chicken globin genes" Proc Natl Acad Sci. USA 85:5976 (1988).
50. Plum M, Frampton J, Wainwright H, Walker M, Macleod K, Goodwin G, and Harrisson P, "GATAAG: A cis-control region binding an erythroid specific nuclear factor with a role in globin and non-globin gene expression" Nucleic Acids Res, 17:73 (1989).
51. Philipsen S, Talbot D, Fraser P, and Grosvelt F, "The β-globin dominant control region: Hypersensitive site 2" EMBO J. 9:2159 (1990).
52. Beaupain D, Elouet JF, and Romeo PH, "Initiation of transcription of erythroid promoter of the porphobilinogen deaminase gene is regulated by a cis-acting sequence around the cap site" Nucleic Acids Res. 18:6509 (1990).
53. Maouche L, Tournamille C, Hattab C, Boffa G, Cartron JP, and Chretian S, "Cloning of the gene encoding the human erythropoietin receptor" Blood 78:2557 (1991).
54. Rahuel C, Vinit M-H, Lemarchandel V, Cartron JP, and Romeo P-H, "Erythroid specific activity of the glycophorin B promoter requires GATA-1 mediated displacement of a repressor" EMBO J. 11:4095 (1992).
55. Cherif-Zahar B, LeVan Kim C, Rouillac C, Raynal V, Cartron JP, and Colin YP, "Organization of the gene (RHCE) encoding the human blood group RhCcEe antigens and characterization of the promoter region" Genomics 19:68 (1994).
56. Crotta S, Nicolis S, Ronchi A, Ottolenghy S, Ruzzi L, Shimada Y, Migliaccio AR, and Migliaccio G, "Progressive inactivation of the expression of the erythroid transcription factor in GM - and G-CSF-dependent cell lines" nucleic Acid Res. 18:6864 (1990).
57. Orkin SH, Tsai SF, Zan LI, Martin D, and Whitelaw E, "The erythroid-specific transcription factor GATA-1: Structure and expression" in Regulation of Hemoglobin Switching, (Stamatoyannopoulus G, Nienhuis AW, eds) Alan R Liss, New York. pp 310 (1992).
58. Crossley M, and Orkin SH, "Regulation of the β globin locus" Current Opinion in Genetics and Development 3:232 (1993).
59. Goosens M and Kan YW, "DNA analysis in the diagnosis of hemoglobin disorders" Methods Enzymol 76:805 (1981).
60. Wahle E and Keller W, "The biochemistry of 3' end cleavage and polyadenylation of messenger RNA precursors" Ann Rev Biochem 61:419 (1992).
61. Rastinejad F and Blau HM, "Genetic complementation reveals a novel regulatory role for 3' untranslated regions in growth and differentiation" Cell 72:903 (1993).
62. Jackson RJ, "Cytoplasmic regulation of mRNA function:The importance of the 3' untranslated region" Cell 74:9 (1993).
63. John, SWM, Weitzner G, Rozen R, and Scriver CR, "A rapid procedure for extracting genomic DNA from leukocytes" Nucleic Acids Res 19:408 (1991).

## Claims

1. A diagnostic method for determining at least one Kell genotype of a subject, wherein said at least one Kell genotype is selected from the group consisting of K6 or K7, and combinations thereof, comprising:
(a) generating a nucleic acid fragment from a nucleic acid sample obtained from a subject, the detection of which permits the differentiation of alleles of a K6/K7 Kell polymorphism by virtue of a T to C base substitution at nt 1910 in exon 17 and/or an A to G base substitution at nt 2019 in exon 17;
(b) detecting said nucleic acid fragment; and
(c) determining the genotype of said subject with respect to said Kell polymorphism based on said detecting.

2. The diagnostic method of claim 1, wherein said generating step comprises selectively cleaving said nucleic acid sample to provide at least one cleavage fragment whose detection permits the differentiation of alleles of a K6/K7 Kell polymorphism and wherein said detecting step comprises detecting said at least one cleavage fragment.

3. The diagnostic method of claim 2, wherein said selectively cleaving step comprises digesting said nucleic acid sample with at least one restriction enzyme which cleaves said nucleic acid sample differentially based on a nucleotide sequence difference at said Kell polymorphism locus.

4. The diagnostic method of claim 3, wherein said nucleic acid sample comprises a Kell K6/K7 polymorphism locus and said at least one restriction enzyme is selected from the group consisting of MnlI, DdeI, and a combination thereof.

5. The diagnostic method of claim 3, wherein said generating step further comprises amplifying a Kell nucleic acid using a Kell allele-nonspecific primer to provide amplified Kell nucleic acid.

6. The diagnostic method of claim 5, wherein said generating step comprises amplifying said Kell nucleic acid prior to said selectively cleaving step.

7. The diagnostic method of claim 5, wherein said generating step comprises amplifying said Kell nucleic acid following said selectively cleaving step.

8. The diagnostic method of claim 5, wherein said amplifying step comprises amplifying nucleic acid by polymerase chain reaction, ligase chain reaction, or a combination thereof.

9. The diagnostic method of claim 1, wherein said generating step comprises:
differentially amplifying said nucleic acid sample using a Kell allele-specific primer to provide said nucleic acid fragment.

10. The diagnostic method of claim 9, wherein said differentially amplifying step comprises differentially amplifying said nucleic acid sample by polymerase chain reaction, ligase chain reaction, or a combination thereof.

11. The diagnostic method of claim 9, wherein said differentially amplifying step comprises amplifying said nucleic acid sample using a Kell allele-specific primer which differentially amplifies alleles of a K6/K7 Kell polymorphism.

12. The diagnostic method of claim 11, wherein said differentially amplifying step comprises amplifying said nucleic acid sample using an allele-specific primer which differentially amplifies alleles of a K6/K7 polymorphism.

13. The diagnostic method of claim 12, wherein said primer includes a primer comprising a nucleotide sequence selected from the group consisting of: CTC ACC TAG GCA GCA CCA ACC CTA (SEQ ID NO: 64) and ITA CCT GGA GGG CAT GGT TGT CAC T (SEQ ID NO: 65).

14. A diagnostic kit for determining a Kell genotype by detecting target nucleic acid sequences specific to Kell alleles, said kit comprising:
(a) a primer set including at least two PCR primers wherein each of the PCR primers is an oligonucleotide that specifically binds to or causes elongation through a sequence specific to one of at least two Kell alleles, wherein the at least two Kell alleles differ at the K6/K7 polymorphism locus by virtue of a T to C base substitution at nt 1910 in exon 17 and/or an A to G base substitution at nt 2019 in exon 17; and
(b) a microtiter plate having a plurality of wells and having bound thereto oligonucleotide capture probes which specifically hybridize to said target sequences.

15. The diagnostic kit according to claim 14, wherein said primer set is CTC ACC TAG GCA GCA CCA ACC CTA (SEQ ID NO: 64) and TTA CCT GGA GGG CAT GGT TGT CAC T (SEQ ID NO: 65).

## Patentansprüche

1. Diagnoseverfahren zum Bestimmen mindestens eines Kell-Genotyps einer Person, wobei der mindestens eine Kell-Genotyp aus der Gruppe ausgewählt wird, die aus K6 oder K7 oder Kombinationen davon besteht, umfassend:
(a) Erzeugen eines Nukleinsäurefragments aus einer Nukleinsäureprobe, die von einer Person erhalten wird, dessen Detektion die Differenzierung von Allelen eines K6/K7-Kell-Polymorphismus vermöge einer Basensubstitution von T zu C bei nt 1910 in exon 17 und/oder einer Basensubstitution von A zu G bei nt 2019 in exon 17 gestattet;
(b) Detektieren des Nukleinsäurefragments; und
(c) Bestimmen des Genotyps der Person in Bezug auf de n Kell-Polymorphismus basierend auf die Detektion.

2. Diagnoseverfahren nach Anspruch 1, wobei der Schritt zum Erzeugen umfasst, die Nukleinsäureprobe selektiv zu spalten, um mindestens ein Spaltfragment bereitzustellen, dessen Detektion die Differenzierung von Allelen eines K6/K7-Kell-Polymorphismus gestattet, und wobei der Schritt zum Detektieren umfasst, das mindestens eine Spaltfragment zu detektieren.

3. Diagnoseverfahren nach Anspruch 2, wobei der Schritt zum selektiven Spalten umfasst, die Nukleinsäurep robe mit mindestens einem Restriktionsenzym auszuschließen, das die Nukleinsäure probe basierend auf einer Nukleotidsequenzdifferenz am de m Kell-Polymorphismuslocus differenziell spaltet.

4. Diagnoseverfahren nach Anspruch 3, wobei die Nukleinsäureprobe einen Kell-K6/K7-Polymorphismuslocus umfasst und das mindestens eine Restriktionsenzym aus der Gruppe ausgewählt wird, die aus MnII, DdeI und einer Kombination davon besteht.

5. Diagnoseverfahren nach Anspruch 3, wobei der Schritt zum Erzeugen weiter umfasst, eine Kell-Nukleinsäure unter Verwendung eines Kell-Allelunspezifischen Primers zu amplifizieren, um amplifizierte Kell-Nukleinsäure bereitzustellen.

6. Diagnoseverfahren nach Anspruch 5, wobei der Schritt zum Erzeugen umfasst, die Kell-Nukleinsäure vor dem Schritt zum selektiven Spalten zu amplifizieren.

7. Diagnoseverfahren nach Anspruch 5, wobei der Schritt zum Erzeugen umfasst, die Kell-Nukleinsäure nach de m Schritt zum selektiven Spalten zu amplifizieren.

8. Diagnoseverfahren nach Anspruch 5, wobei der Schritt zum Amplifizieren umfasst, Nukleinsäure durch Polymerase-Kettenreaktion, Ligase-Kettenreaktion oder eine Kombination davon zu amplifizieren.

9. Diagnoseverfahren nach Anspruch 1, wobei der Schritt zum Erzeugen umfasst:
differenzielles Amplifizieren der Nukleinsäureprobe unter Verwendung eines Kell-Allelspezifischen Primers, um das Nukleinsäurefragment bereitzustellen.

10. Diagnoseverfahren nach Anspruch 9, wobei der Schritt zum differenziellen Amplifizieren umfasst, die Nukleinsäureprobe durch Polymerase-Kettenreaktion, Ligase-Kettenreaktion oder eine Kombination davon differenziell zu amplifizieren.

11. Diagnoseverfahren nach Anspruch 9, wobei der Schritt zum differenziellen Amplifizieren umfasst, die Nukleinsäureprobe unter Verwendung eines Kell-Allelspezifischen Primers, der Allele eines K6/K7-Kell-Polymorphismus differenziell amplifiziert, zu amplifizieren.

12. Diagnoseverfahren nach Anspruch 11, wobei der Schritt zum differenziellen Amplifizieren umfasst, die Nukleinsäureprobe unter Verwendung eines Allelspezifischen Primers, der Allele eines K6/K7-Polymorphismus differenziell amplifiziert, zu amplifizieren.

13. Diagnoseverfahren nach Anspruch 12, wobei der Primer einen Primer enthält, der eine Nukleotidsequenz umfasst, die aus der Gruppe ausgewählt wird, die aus: CTC ACC TAG GCA GCA CCA ACC CTA (SEQ ID NO: 64) und TTA CCT GGA GGG CAT GGT TGT CAC T (SEQ ID NO: 65) besteht.

14. Diagnosesatz zum Bestimmen eines Kell-Genotyps durch Detektieren von Ziel-Nukleinsäuresequenzen, die spezifisch für Kell-Allele sind, der Satz umfassend:
(a) Primersatz, enthaltend mindestens zwei PCR-Primer, wobei jeder der PCR-Primer ein Oligonukleotid ist, das speziell anbindet an oder Längung bewirkt durch eine Sequenz, die für ein von mindestens zwei Kell-Allelen spezifisch ist, wobei die mindestens zwei Kell-Allele sich an de m K6/K7-Polymorphismuslocus vermöge einer Basensubstitution von T zu C bei nt 1910 in exon 17 und/oder einer Basensubstitution von A zu G bei nt 2019 in exon 17 unterscheiden; und
(b) eine Mikrotiterplatte mit einer Vielzahl von Senken und woran Oligonukleotidfängersonden gebunden sind, die speziell an die Zielsequenzen hybridisieren.

15. Diagnosesatz nach Anspruch 14 , wobei der Primersatz CTC ACC TAG GCA GCA CCA ACC CTA (SEQ ID NO: 64) und TTA CCT GGA GGG CAT GGT TGT CAC T (SEQ ID NO: 65) ist.

## Revendications

1. Procédé de diagnostic pour déterminer au moins un génotype de Kell d'un sujet, ledit au moins un génotype de Kell étant sélectionné dans le groupe consistant en K6 ou K7, et des combinaisons de ceux-ci, comprenant :
(a) la génération d'un fragment d'acide nucléique à partir d'un échantillon d'acide nucléique obtenu d'un sujet, dont la détection permet la différenciation d'allèles d'un polymorphisme des gènes K6/K7 de Kell en vertu d'une substitution de base T à C au nt 1910 dans l'exon 17 et/ou une substitution de base A à G au nt 2019 dans l'exon 17 ;
(b) la détection dudit fragment d'acide nucléique ; et
(c) la détermination du génotype dudit sujet relativement audit polymorphisme de Kell en fonction de ladite détection.

2. Procédé de diagnostic selon la revendication 1, dans lequel ladite étape de génération comprend le clivage sélectif dudit échantillon d'acide nucléique pour fournir au moins un fragment de clivage dont la détection permet de différencier les allèles d'un polymorphisme des gènes K6/K7 de Kell et dans lequel ladite étape de détection comprend la détection dudit au moins un fragment de clivage.

3. Procédé de diagnostic selon la revendication 2, dans lequel ladite étape de clivage sélectif comprend la digestion dudit 'échantillon d'acide nucléique avec au moins un enzyme de restriction qui clive ledit échantillon d'acide nucléique différentiellement en fonction d'une différence de séquence nucléotidique audit locus de polymorphisme de Kell.

4. Procédé de diagnostic selon la revendication 3, dans lequel l'échantillon d'acide nucléique comprend un locus de polymorphisme des gènes K67/K7 de Kell et ledit au moins un enzyme de restriction est sélectionné dans le groupe consistant en MnII, DdeI et une combinaison de ceux-ci.

5. Procédé de diagnostic selon la revendication 3, dans lequel ladite étape de génération comprend en outre l'amplification d'un acide nucléique de Kell en utilisant une amorce non spécifique des allèles de Kell pour produire un acide nucléique de Kell amplifié.

6. Procédé de diagnostic selon la revendication 5, dans lequel ladite étape de génération comprend l'amplification dudit acide nucléique de Kell avant ladite étape de clivage sélectif.

7. Procédé de diagnostic selon la revendication 5, dans lequel ladite étape de génération comprend l'amplification dudit acide nucléique de Kell après ladite étape de clivage sélectif.

8. Procédé de diagnostic selon la revendication 5, dans lequel ladite étape d'amplification comprend l'amplification de l'acide nucléique par réaction en chaîne par polymérase, réaction en chaîne par ligase ou une combinaison de celles-ci.

9. Procédé de diagnostic selon la revendication 1, dans lequel ladite étape de génération comprend :
l'amplification différentielle dudit échantillon d'acide nucléique en utilisant une amorce spécifique des allèles de Kell pour produire ledit fragment d'acide nucléique.

10. Procédé de diagnostic selon la revendication 9, dans lequel ladite étape d'amplification différentielle comprend l'amplification différentielle dudit échantillon d'acide nucléique par réaction en chaîne par polymérase, réaction en chaîne par ligase ou une combinaison de celles-ci.

11. Procédé de diagnostic selon la revendication 9, dans lequel ladite étape d'amplification différentielle comprend l'amplification dudit échantillon d'acide nucléique en utilisant une amorce spécifique des allèles de Kell qui amplifie différentiellement les allèles d'un polymorphisme des gènes K6/K7 de Kell.

12. Procédé de diagnostic selon la revendication 11, dans lequel ladite étape d'amplification différentielle comprend l'amplification dudit échantillon d'acide nucléique en utilisant une amorce spécifique des allèles qui amplifie différentiellement les allèles d'un polymorphisme des gènes K6/K7.

13. Procédé de diagnostic selon la revendication 12, dans lequel ladite amorce comporte une amorce comprenant une séquence nucléotidique sélectionnée dans le groupe consistant en : CTC ACC TAG GCA GCA CCA ACC CTA (ID SEQ N° 64) et TTA CCT GGA GGG CAT GGT TGT CAC T (ID SEQ N° 65).

14. Kit de diagnostic pour déterminer un génotype de Kell en détectant des séquences d'acide nucléique cibles spécifiques aux allèles de Kell, ledit kit comprenant :
(a) un ensemble d'amorces comportant au moins deux amorces PCR, chacune des amorces PCR étant un oligonucléotide qui se lie spécifiquement à l'un d'au moins deux allèles de Kell, ou entraîne son allongement par une séquence spécifique à celui-ci, les au moins deux allèles de Kell différant au locus de polymorphisme des gènes K6/K7 en vertu d'une substitution de base T à C au nt 1910 dans l'exon 17 et/ou une substitution de base A à G au nt 2019 dans l'exon 17; et
(b) une plaque microtitre ayant une pluralité de puits et auxquels sont liées des sondes de capture d'oligonucléotides qui s'hybridisent spécifiquement auxdites séquences cibles.

15. Kit de diagnostic selon la revendication 14, dans lequel ledit ensemble d'amorces est CTC ACC TAG GCA GCA CCA ACC CTA (ID SEQ N° 64) et TTA CCT GGA GGG CAT GGT TGT CAC T (ID SEQ N° 65).
